(19) Europäisches Patentamt
European Patent Office
Office européen des brevets

(11) Veröffentlichungsnummer: **0 372 486 B1**

(12) # EUROPÄISCHE PATENTSCHRIFT

(45) Veröffentlichungstag der Patentschrift: **01.06.94**

(21) Anmeldenummer: **89122396.8**

(22) Anmeldetag: **05.12.89**

(51) Int. Cl.5: **C07C 279/14**, A61K 31/195, C07C 237/42, C07K 5/06, C07C 257/18, C07D 211/62, A61K 31/445

(54) **Neue Benzoesäure- und Phenylessigsäurederivate, ihre Herstellung und Verwendung als Heilmittel.**

(30) Priorität: **08.12.88 CH 4543/88**
**11.10.89 CH 3703/89**

(43) Veröffentlichungstag der Anmeldung:
**13.06.90 Patentblatt 90/24**

(45) Bekanntmachung des Hinweises auf die Patenterteilung:
**01.06.94 Patentblatt 94/22**

(84) Benannte Vertragsstaaten:
**AT BE CH DE ES FR GB GR IT LI LU NL SE**

(56) Entgegenhaltungen:
**EP-A- 0 271 099**
**DE-A- 3 700 166**

**THROMBOSIS AND HAEMOSTASIS, Band 56, Nr. 2, 21. Oktober 1986, S. 155-159, F. K. Schattauer Verlag GmbH; Stuttgart, DE; H.-J. KOLDE et al: "New chromogenic substrates for thrombin with increased specificity"**

(73) Patentinhaber: **F. HOFFMANN-LA ROCHE AG**
**Postfach 3255**
**CH-4002 Basel(CH)**

(72) Erfinder: **Alig, Leo, Dr.**
**Liebrütistrasse 32**
**CH-4303 Kaiseraugst(CH)**
Erfinder: **Edenhofer, Albrecht, Dr.**
**Rudolf Wackernagelstrasse 35**
**CH-4125 Riehen(CH)**
Erfinder: **Müller, Marcel, Dr.**
**Ouellenweg 10**
**CH-4402 Frenkendorf(CH)**
Erfinder: **Trzeciak, Arnold**
**Talstrasse 54**
**D-7860 Schopfheim(DE)**
Erfinder: **Weller, Thomas, Dr.**
**Riehenring 71**
**CH-4058 Basel(CH)**

(74) Vertreter: **Lederer, Franz, Dr. et al**
**Lederer, Keller & Riederer**
**Patentanwälte**
**Prinzregentenstrasse 16**
**D-80538 München (DE)**

**EP 0 372 486 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Benzoesäure- und Phenylessigsäurederivate. Verfahren zu deren Herstellung pharmazeutische Präparate, die solche Verbindungen enthalten, sowie die Verwendung dieser Verbindungen bei der Herstellung pharmazeutischer Präparate.

Die Erfindung betrifft insbesondere Benzoesäure- und Phenylessigsäurederivate der Formel

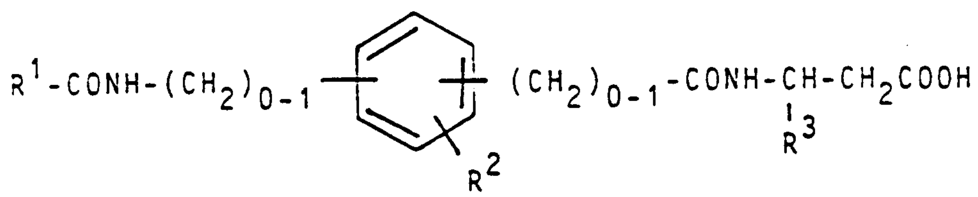

I

worin $R^1$ eine Gruppe der Formel

$-C(H,R^a)-(CH_2)_n-NH-R^b$ (R-1)

$-(T)_{1 \text{ oder } 0}-C_6H_4-CH_2NH-R^c$ (R-2)

$-(T)_{1 \text{ oder } 0}-C_6H_4-(NH)_{1 \text{ oder } 0}-C(NH)NH_2$ (R-3)

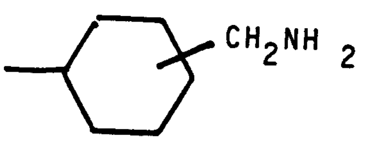

R-4 oder

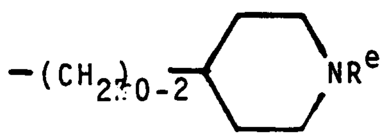

R-5

$R^a$ H, $NH_2$, $-NHCOO-C_{1-4}$-Alkyl, -NH-Z oder $-NHCOCH_2N(Y)-CH_2CH_2NH-Y$, und n 1-6 oder, falls $R^a$ H ist, n auch 0 sein kann,

Y H, Boc oder Z;

$R^b$ H, Amidino oder $-C(NH)-(CH_2)_{0-3}CH_3$;

$R^c$ H oder Amidino;

T $-CH_2-$, $-CH=CH-$ oder $-C(H,R^{d)}-CH_2-$ ist;

$R^d$ die gleiche Bedeutung wie $R^a$ hat oder -NHCO-phenyl, -NHCO-phenylen-$N_3$ oder $-NHSO_2$-Aryl ist;

$R^e$ H oder Amidino;

$R^3$ H, $-CONH_2$, $-COR^f$, $COOR^g$ oder Aryl;

$R^f$ der Rest einer über die Aminogruppe gebundenen $\alpha$-Aminocarbonsäure oder eines Esters oder Amids davon;

$R^g$ H oder nieder-Alkyl;

$R^2$ H, $CH_3$, $OCH_3$, $NO_2$, Halogen, $NH_2$, NHCO-phenylen-COOH, $-NHCO(CH_2)_{1-4}$-COOH, $OR^h$, $-CH_2CH_2OR^h$, $-CH_2CH_2OCH_2CH_2OR^h$, oder $-CH_2COOR^h$; und

$R^h$ H oder nieder-Alkyl ist.

sowie Hydrate oder Solvate und physiologisch verwendbare Salze davon.

Im Rahmen der vorliegenden Erfindung bezeichnet Me Methyl. Bzl Benzyl, tBu t-Butyl, Boc t-Butoxycarbonyl, Z Benzyloxycarbonyl, Arg L-Arginyl, Orn L-Ornithyl, Val L-Valyl, Phe L-Phenylalanyl, Leu L-Leucyl, Ileu L-Isoleucyl, Ser L-Seryl, Thr L-Threonyl, Gly Glycyl, Ala L-Alanyl, Asp L-$\alpha$-Aspartyl.

Aryl bezeichnet mono- oder bicyclische Reste wie Phenyl, Tolyl und $\alpha$- oder $\beta$-Naphthyl.

Beispiele von über die Aminogruppe gebundenen $\alpha$-Aminocarbonsäuren sind Val, Phe, Leu, Ileu, Ser, Thr, N-Isopropyl-Gly, $\beta$-Cyclohexyl-Ala, $\beta$-(1-Naphthyl)-Ala und Cycloleucin

Die Verbindungen der Formel I können solvatisiert, insbesondere hydratisiert sein. Die Hydratisierung kann im Zuge des Herstellungsverfahrens erfolgen oder allmählich als Folge hygroskopischer Eigenschaften einer zunächst wasserfreien Verbindung der Formel I auftreten.

Beispiele von physiologisch verwendbaren Salzen der Verbindungen der Formel I sind Salze mit physiologisch verträglichen Mineralsäuren, wie Salzsäure, Schwefelsäure oder Phosphorsäure, oder mit organischen Säuren, wie Methansulfonsäure, Essigsäure, Trifluoressigsäure, Zitronensäure, Fumarsäure,

Bernsteinsäure oder Salicylsäure. Die Verbindungen der Formel I können auch Salze mit physiologisch verträglichen Basen bilden. Beispiele solcher Salze sind Alkalimetall-, Erdalkalimetall-, Ammonium- und Alkylammoniumsalze, wie das Na-, K-, Ca- oder Trimethylammoniumsalz. Verbindungen der Formel I, die eine Amino, Amidino- oder Guanidinogruppe enthalten, können in Form von Zwitterionen vorliegen.

Die Verbindungen der Formel I, die ein oder mehrere asymmetrische C-Atome enthalten, können als Enantiomere, als Diastereomere oder als Gemische davon, z.B. als Racemate, vorliegen.

Eine bevorzugte Gruppe von Verbindungen der Formel sind Verbindungen der Formel

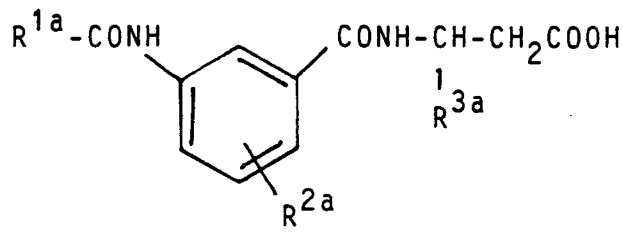

IA

worin $R^{1a}$ eine Gruppe der Formeln R-1, R-2 oder R-3 ist;

$R^{2a}$ H, $-CH_3$, $-OCH_3$ $-NO_2$, Halogen, $-NH_2$, -NHCO-phenylen-COOH oder $-NHCO(CH_2)_{1-4}-COOH$ ist;

$R^{3a}$ H, $-CONH_2$ oder $-COR^i$;

$R^i$ der Rest einer über die Aminogruppe gebundenen α-Aminocarbonsäure ist,

und R-1, R-2 und R-3 die oben angegebene Bedeutung haben.

Bevorzugte Verbindungen der Formel I und IA sind ferner diejenigen, worin $R^1$ eine Gruppe (Amino oder Guanidino)-$(CH_2)_{1-7}$-, α-(Amino oder Guanidino)-(m oder p)-tolyl oder α-Amino-p-tolylmethyl oder worin $R^1$-CO- den Säurerest von Arginin, Ornithin, $N^2$-Boc-Arginin oder $N^2$-Boc-Ornithin darstellt, oder 3-(p-Amidinophenyl)-DL-alanyl oder p-Amidinobenzoyl darstellt.

Bevorzugt sind ferner die Verbindungen der Formel I und IA worin $R^3$ Wasserstoff, Carboxyisobutylidenaminocarbonyl oder 1-Carboxy-2-(1-naphthyl)äthylidenaminocarbonyl ist, sowie diejenigen, worin $R^2$ H, $CH_3$, $OCH_3$, $NO_2$ oder Cl ist.

Die Verbindungen der Formel I können erfindungsgemäss dadurch erhalten werden, dass man aus einer Verbindung der Formel

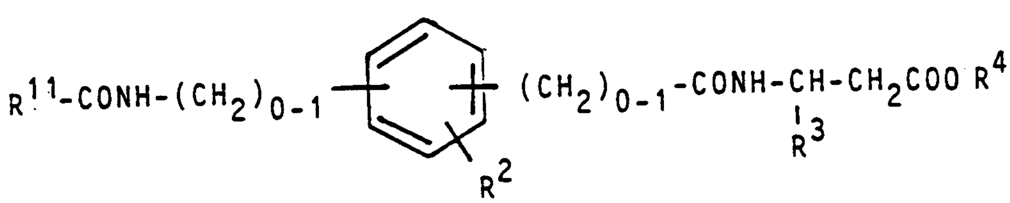

II

worin $R^{11}$ ein Rest der Formeln R-1, R-2, R-3, R-4 oder R-5 gemäss obiger Definition darstelllt, in denen eine gegebenenfalls anwesende Amino-, Amidino- oder Guanidinogruppe in geschützter Form vorliegen kann, $R^4$ Wasserstoff oder eine leicht spaltbare Estergruppe ist und $R^2$ und $R^3$ die weiter oben angegebene Bedeutung haben; und wobei das Molekül mindestens eine leicht spaltbare Estergruppe oder eine geschützte Amino-, Amidino- oder Guanidinogruppe enthält,

Estergruppe(n) und/oder Amino-, Amidino- oder Guanidinoschutzgruppe(n) abspaltet, gewünschtenfalls eine im Rest $R^1$ enthaltene, reaktionsfähige Gruppe funktionell abwandelt, und gewünschtenfalls eine Verbindung der Formel I in ein physiologisch verträgliches Salz überführt oder ein Salz einer Verbindung der Formel I in die freie Säure oder Base überführt.

Beispiele von geschützten Amino-, Amidino- und Guanidinoschutzgruppen sind -NH-Z, -NH-Boc und Azido; -C(NH)NH-Z; $-NHC(NH)NH-NO_2$ und -NHC(N-Boc)-NH-Boc. Beispiele von leicht spaltbaren Estergruppen $R^4$ sind Methyl, t-Butyl und Benzyl. Ein Beispiel für einen in Form eines leicht spaltbaren Esters vorliegenden Rests $R^o$ ist Benzyloxycarbonylisobutylidenamino.

Die Abspaltungen der Estergruppen und der $NH_2$-Schutzgruppen lassen sich in an sich bekannter Weise durchführen. Beispielsweise kann man eine Estergruppe mit einer Base, wie einem Alkalimetallhydroxyd, z.B. Natronlauge, verseifen. Benzylester können durch Hydrierung in Gegenwart eines Edelmetallkatalysators, wie Palladium auf Kohlenstoff (Pd/C) in einem Lösungsmittel, wie Methanol, Athanol, Ameisensäure oder Essigsäure, bei einer Temperatur bis zu etwa 40 °C, vorzugsweise bei Raumtemperatur gespalten werden. Dabei werden gleichzeitig in der Gruppe R enthaltene Amino- oder Amidinoschutzgruppen, wie Z; bzw. Guanidinoschutzgruppen, wie $NO_2$, abgespalten.

Eine im Substituenten $R^{11}$ enthaltene geschützte Aminogruppe, z.B. -NH-Boc, lässt sich z.B. mit einer Säure, wie Ameisensäure oder Trifluoressigsäure, bei einer Temperatur bis zu 40 °C, vorzugsweise bei etwa Raumtemperatur spalten. Dabei werden gleichzeitig Estergruppen $R^3$, z.B. t-Butyl, bzw. Amidinoschutzgruppen, wie Boc, abgespalten.

Die funktionellen Abwandlungen reaktionsfähiger Gruppen in $R^1$ lassen sich ebenfalls nach an sich geläufigen Methoden bewerkstelligen. So kann man eine in dem Substituenten $R^1$ enthaltene primäre Aminogruppe -NH-$R^b$ oder -NH-$R^c$ in eine Guanidinogruppe umwandeln, z.B. mittels 2-[(Aminoiminomethyl)-thio]äthansulfonsäure in Gegenwart einer Base, wie Natriumcarbonat oder Natriumhydroxid, bei einer Temperatur bis zu 40 °C, vorzugsweise bei etwa Raumtemperatur.

Eine in einem Substituenten $R^1$ enthaltene primäre Aminogruppe $R^a$ kann man in die -NH-Boc-Gruppe umwandeln, z.B. mittels Di-t-Butyl-dicarbonat in einem Lösungsmittel. wie Dioxan, in Gegenwart von Pyridinhydrobromid und Natriumbicarbonat bei einer Temperatur bis zu 40 °C, vorzugsweise bei etwa Raumtemperatur.

Umgekehrt lässt sich eine im Substituenten $R^1$ enthaltene geschützte Aminogruppe $R^a$, wie -NH-Boc, abspalten, z.B. mittels Ameisensäure, wie oben im Zusammenhang mit der Abspaltung von Schutzgruppen beschrieben.

Die Verbindungen der Formel II sind neu und ebenfalls Gegenstand der vorliegenden Erfindung. Ihre Herstellung kann ausgehend von bekannten Verbindungen nach an sich bekannten und jedem Fachmann geläufigen Methoden erfolgen.

So lassen sich Verbindungen der Formel II dadurch erhalten, dass man eine Säure der Formel

$R^{12}$-COOH III

mit einem Amin der Formel

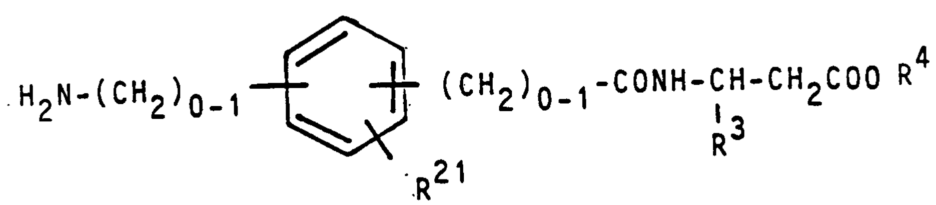

IV

oder eine Säure der Formel

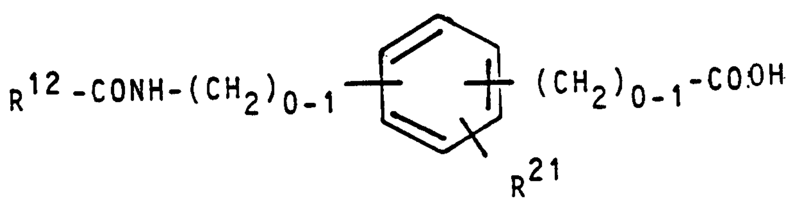

V

mit einem Amin der Formel

$H_2N$-CH($R^3$)-$CH_2COOR^4$ VI

worin $R^3$ und $R^4$ die obige Bedeutung haben und $R^{12}$ und $R^{21}$ einen Rest $R^1$ bzw. $R^2$ darstellt, in dem eine gegebenenfalls anwesende Amino-, Amidino- oder Guanidinogruppe in geschützter Form vorliegt, unter Bildung einer Amidbindung kuppelt. Aus dem so erhaltenen Reaktionsprodukt können sodann darin enthaltene Estergruppen und Amino-, Amidino- oder Guanidinoschutzgruppen gegebenenfalls selektiv abgespalten werden. Die Kupplungsreaktionen in den Säuren der Formeln III und V mit den Aminen der Formeln IV und VI können nach aus der Peptidchemie an sich bekannten Methoden durchgeführt werden

Eine Säure der Formel III kann man z.B. mit Chlorameisensäureisobutylester und Triäthylamin in

Tetrahydrofuran (THF) bei einer Temperatur zwischen etwa -10 °C und Raumtemperatur aktivieren und mit dem Trifluoracetat einer Base der Formel IV bei der besagten Temperatur reagieren lassen.

Eine Säure der Formel V kann man z.B. in THF mit 2-Chlor-4,6-dimethoxy-1,3,5-triazin und N-Methylmorpholin aktivieren und mit dem p-Toluolsulfonat eines Amins der Formel VI reagieren lassen. Man kann auch die Säure V in Dimethylformamid (DMF) mit Chlorameisensäureisobutylester und N-Methylmorpholin aktivieren und bei einer Temperatur zwischen etwa -5 °C und Raumtemperatur mit dem Trifluoracetat des Amins VI reagieren lassen.

Eine in einem Ester der Formel II enthaltene Estergruppe z.B. Methoxycarbonyl, lässt sich selektiv spalten, z.B. durch Verseifung in methanolischer Natronlauge. Eine im Ester II vorhandene Aminoschutzgruppe, wie Boc, bleibt dabei erhalten.

Geschützte Amidinogruppen, die als Vorläufer der Reste R-1, R-2 und R-3 vorliegen können, können aus entsprechenden Nitrilen hergestellt werden durch Umsetzung mit Schwefelwasserstoff und Triäthylamin in Pyridin zum Thioamid, Methylierung mit Methyljodid und anschliessende Umsetzung mit Ammoniumacetat in Methanol. Die Schutzgruppe kann dann durch Behandlung des Amidins mit Chlorameisensäurebenzylester und Triäthylamin in THF eingeführt werden. Eine geschützte Guanidinogruppe kann aus einer Aminogruppe durch Umsetzung mit N,N'-Bis(tert-butoxycarbonyl)-S-methylisothioharnstoff in tert. Butanol und Wasser in Gegenwart von Triäthylamin aufgebaut werden.

Die Amine der Formel IV lassen sich in an sich bekannter Weise ausgehend von einer Säure der Formel

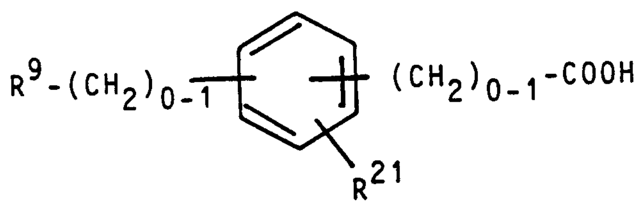

VII

worin $R^9$ eine geschützte Aminogruppe, wie -NH-Boc ist,
und einem Amin der Formel VI herstellen; und die Säuren der Formel V ausgehend von einer Säure der Formel III und einer Verbindung der Formel

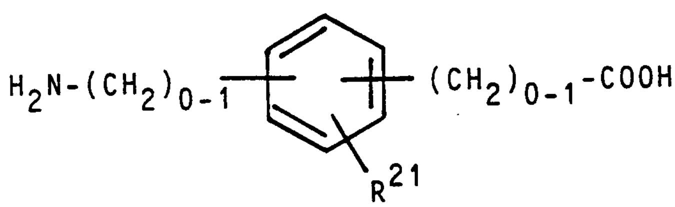

VIII

herstellen, wobei wie oben für die Umsetzung der Verbindungen III und V mit den Verbindungen IV und VI beschrieben vorgegangen werden kann. Amine der Formel IV können auch dadurch hergestellt werden, dass man eine Säure der Formel VII mit einem Amin der Formel

$H_2NCH(COOCH_3)CH_2COOR^4$ IX

zu einer Verbindung der Formel

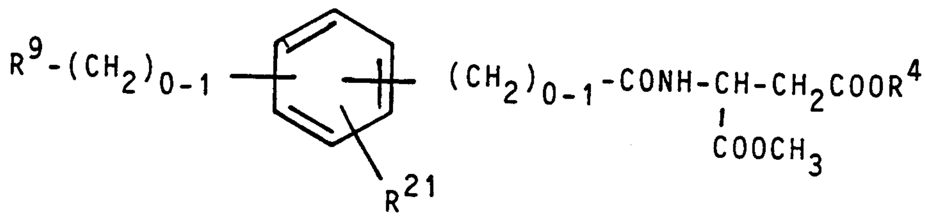

X

umsetzt, die Gruppe $-COOCH_3$ verseift und die erhaltene Säure mit einem Amin $HR^8$ umsetzt.

Darüberhinaus enthalten zahlreiche der nachfolgenden Beispiele ausführliche Angaben betreffend die Herstellung von spezifischen Verbindungen der Formel II. Die Ausgangsprodukte der Formeln III, VI, VIII, VIII-1, IX und XII sind bekannt, z.B. aus J. Med. Chem. 13 (1970) 352; Nippon Kagaku Zasshi 78 (1957) 1768 oder DOS 3 700 166, oder nach an sich bekannten und dem Fachmann geläufigen Methoden herstellbar.

Die Verbindungen der Formel I, ihre Solvate und ihre Salze hemmen sowohl die Bindung von Fibrinogen, Fibronectin und des Willebrand-Faktors an den Fibrinogenrezeptor der Blutplättchen (Glykoprotein IIb/IIIa) als auch die Bindung derselben und weiterer adhäsiver Proteine, wie Vitronectin, Kollagen und Laminin, an die entsprechenden Rezeptoren auf der Oberfläche verschiedener Zelltypen. Die besagten Verbindungen beeinflussen somit Zell-Zell- und Zell-Matrix-Wechselwirkungen. Sie verhindern insbesondere die Entstehung von Blutplättchenthromben und können bei der Bekämpfung bzw. Verhütung von Krankheiten wie Thrombose, Hirnschlag, Herzinfarkt, Entzündung und Arteriosklerose verwendet werden. Ferner haben diese Verbindungen einen Effekt auf Tumorzellen, indem sie deren Metastasierung hemmen. Somit können sie auch als Antitumor-Mittel eingesetzt werden.

Die Hemmung der Fibrinogenbindung an dem Fibrinogenrezeptor, Glykoprotein IIb/IIIa, kann wie folgt nachgewiesen werden:

Das Glykoprotein IIb/IIIa wird aus Triton X-100 Extrakten von menschlichen Blutplättchen gewonnen und durch Lectin-Affinitätschromatographie (Analytical Biochemistry 151, 1985, 169-177) und Chromatographie an einer Arg-Gly-Asp-Ser-Affinitätssäule (Science 231, 1986, 1559-62) gereinigt. Das so erhaltene Rezeptorprotein wird an Mikrotiterplatten gebunden. Die spezifische Bindung von Fibrinogen an den immobilisierten Rezeptor wird mit Hilfe eines ELISA-Systems ("enzyme-linked immunosorbent assay") bestimmt. Die nachstehenden $IC_{50}$-Werte entsprechen derjenigen Konzentration der Testsubstanz, welche benötigt wird, um die Bindung von Fibrinogen an den immobilisierten Rezeptor um 50% zu hemmen:

| Produkt von Beispiel: | 5 | 8 | 9 | 10 | 13 | 14 | 15 | 17 |
|---|---|---|---|---|---|---|---|---|
| $IC_{50}$ ($\mu$M) | 0,27 | 0,16 | 0,24 | 0,26 | 0,22 | 0,3 | 0,08 | 0,38 |

| Produkt von Beispiel: | 21 | 22 | 24 | 28 | 32 | 34 | 35 | 36 |
|---|---|---|---|---|---|---|---|---|
| $IC_{50}$ ($\mu$M) | 0,46 | 0,25 | 0,38 | 0,14 | 0,12 | 0,046 | 0,0001 | 0,27 |

| Produkt von Beispiel: | 41 | 46 | 47 | 51 | 52 | 53 |
|---|---|---|---|---|---|---|
| $IC_{50}$ ($\mu$M) | 0,28 | 0,15 | 0,18 | 0,017 | 0,14 | 0,07 |

| Produkt von Beispiel: | 54 | 55 | 56 | 60 | 64 | 65 |
|---|---|---|---|---|---|---|
| $IC_{50}$ ($\mu$M) | 0,01 | 0,025 | 0,005 | 0,05 | 0,22 | 0,084 |

Die Verbindungen der Formel I sind untoxisch. So hat das Produkt von Beispiel 10 eine $LD_{50}$ von mehr als 1000 mg/kg intraperitoneal und mehr als 2000 mg/kg peroral an der Maus.

Wie eingangs erwähnt, sind Arzneimittel, enthaltend ein m-Aminobenzoat der Formel I, ein Solvat davon oder ein Salz davon, ebenfalls Gegenstand der vorliegenden Erfindung, weiterhin auch ein Verfahren zur Herstellung derartiger Arzneimittel, welches dadurch gekennzeichnet ist, dass man eine oder mehrere der besagten Verbindungen und erwünschtenfalls einen oder mehrere andere therapeutisch wertvolle Stoffe in eine galenische Darreichungsform bringt. Die Arzneimittel können enteral, z.B. oral in Form von Tabletten, Lacktabletten, Dragées, Hart- und Weichgelatinekapseln, Lösungen, Emulsionen oder Suspensionen, oder rektal, z.B. in Form von Suppositorien, oder als Spray verabreicht werden. Die Verabreichung kann aber auch parenteral, z.B. in Form von Injektionslösungen, erfolgen.

Zur Herstellung von Tabletten, Lacktabletten, Dragées und Hartgelatinekapseln kann der Wirkstoff mit pharmazeutisch inerten, anorganischen oder organischen Excipientien vermischt werden. Als solche Excipientien kann man für Tabletten, Dragées und Hartgelatinekapseln z.B. Lactose, Maisstärke oder Derivate davon, Talk, Stearinsäure oder deren Salze, verwenden. Für Weichgelatinekapseln eignen sich als Excipientien z.B. vegetabile Oele, Wachse, Fette, halbfeste und flüssige Polyole; je nach Beschaffenheit des Wirkstoffes sind jedoch bei Weichgelatinekapseln überhaupt keine Excipientien erforderlich. Zur Herstellung von Lösungen und Sirupen eignen sich als Excipientien z.B. Wasser, Polyole, Saccharose, Invertzucker und Glukose, für Injektionslösungen eignen sich z.B. Wasser, Alkohole, Polyole, Glycerin und vegetabile Oele, und für Suppositorien eignen sich z.B. natürliche oder gehärtete Oele, Wachse, Fette und halbflüssige oder flüssige Polyole. Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten.

Für die Bekämpfung bzw. Verhütung der weiter oben genannten Krankheiten kann die Dosierung des Wirkstoffes innerhalb weiter Grenzen variieren und ist natürlich in jedem einzelnen Fall den individuellen Gegebenheiten anzupassen. Im allgemeinen dürfte bei oraler Verabreichung eine Dosis von etwa 0,1 bis 20 mg/kg, vorzugsweise von etwa 0,5 bis 4 mg/kg, pro Tag für den Erwachsenen angemessen sein, wobei aber die soeben angegebene obere Grenze auch überschritten werden kann, wenn sich dies als angezeigt erweisen sollte.

Beispiel 1

900 mg N-[m-[[$N_2$-[(Benzyloxy)carbonyl]-$N_5$-(N-nitroamidino) -L-ornithyl]amino]benzoyl]-$\beta$-alanin-benzylester und 300 mg Pd/C (5%) werden in 20 ml Ameisensäure 18 Stunden unter Wasserstoff gerührt. Das Reaktionsgemisch wird filtriert und das Filtrat im Vakuum eingedampft. Der Rückstand wird in Wasser aufgenommen und im Vakuum wieder eingedampft. Nach Trocknen erhält man 545 mg N-[m-(L-Arginylamino)benzoyl]-$\beta$-alaninformiat (2:3), $[\alpha]_D^{20}$ = +39° ($H_2O$, c = 0,5%).

Zur Herstellung des Ausgangsesters wird m-(1-t-Butoxyformamido)benzoesäure in THF mit 2-Chlor-4,6-dimethoxy-1,3,5-triazin und N-Methylmorpholin aktiviert (2 Stunden, 0°C) und mit $\beta$-Alaninbenzylester-p-toluolsulfonat und N-Methylmorpholin zu N-[m-(1-t-Butoxyformamido)benzoyl]-$\beta$-alaninbenzylester umsetzt, Smp. 130-131°C. Spaltung mit Trifluoressigsäure gibt N-(m-Aminobenzoyl)-$\beta$-alaninbenzylester-trifluoracetat. $N_\alpha$-Z-N-$_\omega$-Nitro-L-arginin wird mit Chlorameisensäureisobutylester und Triäthylamin in THF aktiviert (4 Minuten, -10°C) und mit N-(m-Aminobenzoyl)-$\beta$-alaninbenzylester-trifluoracetat und Triäthylamin zum erwünschten Benzylester gekoppelt, Smp. 157-160°C.

Beispiel 2

N-[m-(L-Arginylamino)benzoyl]-$\beta$-alaninformiat wird in Wasser an Amberlite IR-120 ($H^+$), einem stark sauren $SO_3H$-Gruppen enthaltenden Kationenaustauscherharz, adsorbiert und mit ionenfreiem Wasser neutral gewaschen. Mit wässrigem Ammoniak (2%) wird anschliessend freies N-[m-(L-Arginylamino)-benzoyl]-$\beta$-alanin eluiert, Smp. 93-95°C.

Beispiel 3

390 mg N-[3-[[$N_2$-(Benzyloxycarbonyl)-$N_5$-(N-nitroamidino) -L-ornithyl]amino]-p-anisoyl]-$\beta$-alaninbenzylester und 160 mg Pd/C (5%) werden in 8 ml Ameisensäure 5 Stunden unter Wasserstoff gerührt. Die filtrierte Lösung wird eingedampft und der Rückstand mit Methanol-Ameisensäure (99:1) an Kieselgel gereinigt. Man erhält 195 mg N-[3-(L-Arginylamino)-p-anisoyl]-$\beta$-alaninformiat (1:2), $[\alpha]_D^{20}$ = +46,6° ($H_2O$, c = 0,5%).

Zur Herstellung des Ausgangsesters wird $N_\alpha$-Z-$N_\omega$-Nitro-L-arginin in THF mit 2-Chlor-4,6-dimethoxy-1,3,5-triazin und N-Methylmorpholin aktiviert und mit 3-Amino-4-methoxybenzoesäure und N-Methylmorpholin zu 3-[[$N_2$-(Benzyloxycarbonyl)-$N_5$-(N-nitroamidino) -L-ornithyl]amino]-p-anissäure umgesetzt, Smp. 208-209°C. Diese wird in analoger Weise mit $\beta$-Alaninbenzylester-p-toluolsulfonat zum erwünschten Ester gekoppelt, Smp. 151-153°C.

Beispiel 4

Analog Beispiel 1 erhält man aus 415 mg N-[3-[[$N_2$-(Benzyloxycarbonyl)-$N_5$-(N-nitroamidino) -L-ornithyl]amino]-p-toluoyl]-$\beta$-alaninbenzylester, 264 mg N-[3-(L-Arginylamino)-p-toluoyl]-$\beta$-alaninformiat (2:3), $[\alpha]_D^{20}$ = +18,8° (MeOH, c = 0,5%).

Der Ausgangsester (Smp. 154-156 °C) wird analog Beispiel 3 aus $N_\alpha$-Z-$N_\omega$-Nitro-L-arginin und 3-Amino-4-methylbenzoesäure, via m-[[$N_2$-(Benzyloxycarbonyl)-$N_5$-(N-nitroamidino) -L-ornithyl]amino]-p-toluylsäure, Smp. 235 °C (Zers.) erhalten.

Beispiel 5

545 mg N-[m-[6-[1-(Benzyloxy)formamido]hexanamido]benzoyl] -β-alaninbenzylester und 136 mg Pd/C werden in 10 ml Essigsäure 3 Stunden unter Wasserstoff gerührt. Die filtrierte Lösung wird im Vakuum eingedampft und der Rückstand in Wasser gelöst und wieder eingedampft. Man erhält 330 mg N-[m-(6-Aminohexanamido)benzoyl]-β-alanin, Smp. 221 °C.

Der Ausgangsester (Smp. 121-122 °C) wird analog obigen Beispielen erhalten aus 6-[1-(Benzyloxy)-formamido]hexansäure und N-(m-Aminobenzoyl)-β-alaninbenzylester-trifluoracetat.

Beispiel 6

Analog Beispiel 5 erhält man aus N-[m-[[N-(Benzyloxycarbonyl)-β-alanyl]amino]benzoyl] -β-alaninbenzylester, Smp. 155-156 °; das N-[m-(β-Alanylamino)benzoyl]-β-alanin, Smp. 238 °C.

Beispiel 7

Analog Beispiel 5 erhält man aus N-[m-[8-[1-(Benzyloxy)formamido]octanamido]benzoyl] -β-alaninbenzylester das N-[m-(8-Aminooctanamido)benzoyl]-β-alanin, Smp. 231-233 °C.

Den Ausgangsester (Smp. 123-124 °C) erhält man, in dem man 8-[1-(Benzyloxy)formamidooctansäure und 3-Aminobenzoesäure zu m-[8-[1-(Benzyloxy)formamido]octanamido]benzoesäure, Smp. 167-168 °C koppelt und letztere mit β-Alaninbenzylester-p-toluolsulfonat umsetzt.

Beispiel 8

Analog obigen Beispielen erhält man aus m-[α-[1-(Benzyloxy)formamido]-p-toluamido]benzoesäure, Smp. 250 °, via N-[m[α-[1-(Benzyloxy)formamido]-p-toluamido]benzoyl] -β-alaninbenzylester, Smp. 155-156 °, das N-[m-(α-Amino-p-toluamido)benzoyl]-β-alanin, Smp. 232 °C.

Beispiel 9

Analog obigen Beispielen erhält man aus m-[5-[1-(Benzyloxy)formamido]valeramido]benzoesäure, Smp. 217-221 °, via N-[m-[5-[1-(Benzyloxy)formamido]valeramido]benzoyl]-β-alaninbenzylester, Smp 122-126 °, das N-[m-(5-Aminovaleramido)benzoyl]-β-alanin, Smp. 192-194 °C.

Beispiel 10

439 mg N-[m-(6-Aminohexanamido)benzoyl]-β-alanin (Beispiel 5) und 378 mg 2-[(Aminoiminomethyl)-thio]äthansulfonsäure werden in 1,4 ml gesättigter Natriumcarbonatlösung 22 Stunden bei 20 °C gerührt. Das ausgefallene N-[m-(6-Guanidinohexanamido)benzoyl]-β-alanin wird abgenutscht, mit Wasser gewaschen und durch Kristallisation gereinigt, Smp. 248 °C.

Beispiel 11

Ebenso wird aus N-[m-(8-Aminooctanamido)benzoyl-β-alanin (Beispiel 7) das N-(m-(8-Guanidinooctanamido)benzoyl]-β-alanin, Smp. 221-226 °C, hergestellt.

Beispiel 12

Ebenso wird aus N-[m-(β-Alanylamino)benzoyl]-β-alanin (Beispiel 6) das N-[m-[(N-Amidino-β-alanyl)-amino]benzoyl]-β-alanin, Smp. 241 °, erhalten.

Beispiel 13

Analog wird aus N-[m-(α-Amino-p-toluamido)benzoyl]-β-alanin (Beispiel 8) das N-[m-(α-Guanidino-p-toluamido)benzoyl]-β-alanin, Smp. 221 °C, hergestellt.

Beispiel 14

750 mg [m-[Z-Arg($NO_2$)-NH]-Benzoyl]-Asp(OBzl)-Val-OBzl werden in 50 ml 90% Eisessig gelöst und in Gegenwart von Pd/C (10%) bei Raumtemperatur hydriert. Nach 2 Stunden ist die Hydrierung beendet. Der Katalysator wird abfiltriert und das Filtrat lyophilisiert. Man erhält 490 mg N-[N-[m-(L-Arginylamino)benzoyl]-L-α-aspartyl]-L-valinacetat (1:1), MS: 508 $(M+H)^+$.

Den Ausgangsbenzylester kann man wie folgt herstellen:

a) Eine Lösung von 7,07 g Z-Arg($NO_2$)-OH in 50 ml DMF wird bei -5 °C unter Rühren mit 2,2 ml N-Methylmorpholin, 2,61 ml Chlorameisensäureisobutylester und dann mit einer Lösung von 2,74 g 3-Aminobenzoesäure und 2,2 ml N-Methylmorpholin in 40 ml DMF versetzt. Das Reaktionsgemisch wird 30 Minuten bei -5 °C und 2 Stunden bei Raumtemperatur gerührt; anschliessend zwischen Aethylacetat und 5% $KHSO_4$/10% $K_2SO_4$-Lösung verteilt. Die organische Phase wird mit gesättigter NaCl-Lösung gewaschen und über $Na_2SO_4$ getrocknet. Das Filtrat wird konzentriert, die weissen Kristalle werden abgenutscht und im Vakuum getrocknet. Man erhält 3,0 g m-[[$N_2$-(Benzyloxycarbonyl)-$N_5$-($N_1$-nitroamidino)-L-ornithyl]amino]benzoesäure, Smp. 232 °C; $[\alpha]_D^{20}$ = +5,9° (c = 1, DMF).

b) Eine Lösung von 1,42 g der in a) erhaltenen Säure in 15 ml DMF wird nacheinander bei -5 °C mit 0,33 ml N-Methylmorpholin und 0,39 ml Chlorameisensäureisobutylester und dann bei -10 °C mit einer Lösung von 1,58 g H-Asp(OBzl)-Val-OBzl.$CF_3COOH$ und 0,33 ml N-Methylmorpholin in 10 ml DMF versetzt. Das Reaktionsgemisch wird 10 Minuten bei -5 °C und 2 Stunden bei Raumtemperatur gerührt und anschliessend zwischen Aethylacetat und Wasser verteilt. Die organische Phase wird mit 5% $KHSO_4$/10% $K_2SO_4$ Lösung, Wasser, gesättigter $NaHCO_3$-Lösung, Wasser und gesättigter NaCl-Lösung gewaschen und über $Na_2SO_4$ getrocknet. Das Trockenmittel wird abfiltriert und das Filtrat im Vakuum eingeengt. Der Rückstand wird aus Aether kristallisiert. Man erhält 1,6 g [m-[Z-Arg(NO2)-NH]-Benzoyl]-Asp(OBzl)-Val-OBzl, Smp. 80 °C; $[\alpha]_D^{20}$ = -15.3° (c = 1, DMF).

Beispiel 15

Eine Lösung von 142 mg N-[N-[m-(L-Arginylamino)benzoyl]-L-α-aspartyl]-L-valinacetat (Beispiel 1) in 1 ml Dioxan und 1 ml $H_2O$ wird nacheinander mit 40 mg Pyridin•HBr, 105 mg $NaHCO_3$ und 76 mg Di-t-butyl-dicarbonat versetzt. Nach 3-stündigem Schütteln bei Raumtemperatur wird das Reaktionsgemisch mit Eisessig auf pH 4 angesäuert und über Sephadex G-25S, einem Polysaccharidharz, in 0,2N Essigsäure gereinigt. Die Hauptfraktion wird lyophilisiert und mittels HPLC über Lichrosorb RP18, einem chemisch modifizierten Kieselgel, mit 0,05M Ammoniumacetat und Aethanol gereinigt. Die Hauptfraktion wird aus Wasser lyophilisiert. Man erhält 54 mg [m-(Boc-Arg-NH)Benzoyl]-Asp-Val, MS: 608 $(M+H)^+$.

Beispiel 16

807 mg N-[3-[6-(1-t-Butoxyformamido)hexanamido]-5-nitrobenzoyl] -β-alanin werden in 2,7 ml Ameisensäure 18 Stunden bei Raumtemperatur stehen gelassen. Das Lösungsmittel wird im Vakuum abgedampft. Der Rückstand wird in Wasser gelöst und die Lösung im Vakuum eingedampft. Man erhält 573 mg N-[3-(6-Aminohexanamido)-5-nitrobenzoyl]-β-alaninformiat (2:1), MS: 367 (67, M+H).

Zur Herstellung des Ausgangsmaterials, MS: 367 (N-BOC+H), werden 6-(1-t-Butoxyformamido)-hexansäure und 3-Amino-5-nitrobenzoesäure zur 3-[6-(1-t-Butoxyformamido)hexanamido]-5-nitrobenzoesäure, MS: 396 (2, M+H), und letztere mit β-Alaninmethylester zum N-[3-[6-(1-t-Butoxyformamido)-hexanamido]-5-nitrobenzoyl] -β-alaninmethylester umgesetzt, MS: 381 (100, M-Boc+H). Dieser wird dann in methanolischer Natronlauge verseift.

Beispiel 17

Analog Beispiel 16 wird aus N-[5-[6-(1-tert-Butoxyformamido)hexanamido]-2-chlorbenzoyl -β-alanin das N-[5-(6-Aminohexanamido)-2-chlorbenzoyl]-β-alanin, Smp. 264° aus $H_2O$, erhalten.

Zur Herstellung des Ausgangsmaterials [MS: 456 (1, M+H)] werden 6-(1-t-Butoxyformamido)-hexansäure und 5-Amino-2-chlorbenzoesäure zur 5-[6-(1-tert-Butoxyformamido)hexanamido]-2-chlorbenzoesäure, MS: 385 (2, M+H), letztere mit β-Alaninmethylester zum N-[5-[6-(1-tert-Butoxyformamido)-hexanamido]-2-chlorbenzoyl -β-alaninmethylester gekoppelt. MS: 470 (14, M+H), und dieser dann in methanolischer Natronlauge verseift.

Beispiel 18

Analog Beispiel 16 wird aus N-[5-[3-(1-t-Butoxyformamido)propionamido]-2-nitrobenzoyl] -β-alanin-t-butylester das N-[5-(β-Alanylamino)-2-nitrobenzoyl]-β-alanin erhalten, MS: 325 (77, M+H).

Zur Herstellung des Ausgangsesters werden N-(t-Butoxycarbonyl)-β-alanin und 5-Amino-2-nitrobenzoesäure zur 5-[3-(1-t-Butoxyformamido)propionamido]-2-nitrobenzoesäure gekoppelt, MS: 354 (17, M+H) und letztere mit β-Alanin-t-butylesterumgesetzt.

Beispiel 19

Analog Beispiel 16 wird aus N-[3-[6-[2,3-Bis(t-butoxycarbonyl)guanidino]hexanamido]-5-nitrobenzoyl]-β-alanin das N-[3-(6-Guanidinohexanamido)-5-nitrobenzoyl]-β-alanin hergestellt, MS: 409 (100, M+H).

Zur Herstellung des Ausgangsmaterials, MS: 509 (2, M-Boc+H), wird N-[3-(6-Aminohexanamido)-5-nitrobenzoyl]-β-alanin (Beispiel 16) in t-Butanol, Wasser und Triäthylamin bei 60°C mit N,N′-Bis(t-butoxycarbonyl)-S-methylisothioharnstoff umgesetzt.

Beispiel 20

Analog Beispiel 16 wird aus N-[5-[6-[2,3-Bis(tert-butoxycarbonyl)guanidino]hexanamido] -2-chlorbenzoyl]-β-alanin das N-[5-(6-Guanidinohexanamido)-2-chlorbenzoyl]-β-alanin erhalten, Smp. 235°C.

Das Ausgangsmaterial, MS: 598 (3, M+H), wird aus N-[5-(6-Aminohexanamido)-2-chlorbenzoyl]-β-alanin (Beispiel 17) und N,N′-Bis(t-butoxycarbonyl)-S-methylisothioharnstoff erhalten.

Beispiel 21

1,43 g N-[m-[7-(1-t-Butoxyformamido)heptanamido]benzoyl]-β-alaninbenzylester und 476 mg Pd/C werden in 28 ml Ameisensäure 5 Stunden unter Wasserstoff gerührt. Das Reaktionsgemisch wird filtriert und das Filtrat im Vakuum eingedampft. Der Rückstand wird in Wasser aufgenommen und eingedampft. Aus Methanol erhält man 464 mg N-[m-(7-Aminoheptanamido)benzoyl-β-alanin, Smp. 236°C.

Das Ausgangsmaterial, Smp. 128-129°C (Essigester), wird aus 7-(1-t-Butoxyformamido)heptansäure und N-(m-Aminobenzoyl)-β-alaninbenzylester-trifluoracetat erhalten.

Beispiel 22

Analog Beispiel 10 erhält man aus N-[m-(5-Aminovaleramido)benzoyl]-β-alanin (Beispiel 9) das N-[m-(5-Guanidinovaleramido)benzoyl]-β-alanin, Smp. 167-173°.

Beispiel 23

Ebenso wird aus N-[m(7-Aminoheptanamido)benzoyl]-β-alanin (Beispiel 21) das N-[m-(7-Guanidinoheptanamido)benzoyl]-β-alanin, Smp. 237°C erhalten.

Beispiel 24

Ebenso wird aus N-[m-(4-Aminobutyramido)benzoyl]-β-alanin (Beispiel 25) das N-[m-(4-Guanidinobutyramido)benzoyl]-β-alanin, Smp. >260°C erhalten.

Beispiel 25

Analog Beispiel 5 wird aus N-[m-[4-[1-(Benzyloxy)formamido]butyramido]benzoyl] -β-alaninbenzylester das N-[m-(4-Aminobutyramido)benzoyl]-β-alanin, Smp. 205-206°C. erhalten.

Zur Herstellung des Ausgangsesters, Smp. 119-120°C (aus Aethanol), wird 4-[1-(Benzyloxy)-formamido]buttersäure mit m-Aminobenzoesäure zur m-[4-[1-(Benzyloxy)formamido]-butyramidobenzoesäure und letztere mit β-Alaninbenzylester umgesetzt.

Beispiel 26

Ebenso wird aus N-[m-[α-[1-(Benzyloxy)formamido]-m-toluamido]benzoyl] -β-alaninbenzylester das N-[m-(α-Amino-m-toluamido)benzoyl]-β-alanin, Smp. 242 °C (aus Wasser), erhalten.

Der Ausgangsester, Smp. 151-152 °C (aus Aethanol), wird aus α-[1-(Benzyloxy)formamido]-m-toluylsäure und N-(m-Aminobenzoyl)-β-alaninbenzylester-trifluoracetat hergestellt.

Beispiel 27

Ebenso wird aus N-[m-[[N-[(Benzyloxy)carbonyl] -3-[p-[N-(benzyloxycarbonyl)amidino]phenyl] -DL-alanyl]amino]benzoyl]-β-alaninbenzylester das N-[m-[[3-(p-Amidinophenyl)-DL-alanyl]amino]benzoyl] -β-alaninacetat (1:1) hergestellt, MS: 398 (72, M+H).

Zur Herstellung des Ausgangsesters, MS: 756 (15, M+H) werden N-(Benzyloxycarbonyl)-3-(p-cyanophenyl)-DL-alanin und N-(m-Aminobenzoyl)-β-alaninbenzylester-trifluoracetat zum N-[m-[[N-(Benzyloxycarbonyl)-3-(p-cyanophenyl) -DL-alanyl]amino]benzoyl]-β-alaninbenzylester gekoppelt, MS: 605 (15, M+H), und dieser mit Schwefelwasserstoff und Triäthylamin in Pyridin in den N-[m-[[N-(Benzyloxycarbonyl)-3-[p-(thiocarbamoyl)phenyl] -DL-alanyl]amino]benzoyl]-β-alaninbenzylester, Smp. 145-146 °C (aus Essigester) übergeführt. Letzterer wird dann mit Methyljodid in Aceton und anschliessend mit Ammoniumacetat in Methanol umgesetzt und mit Chlorameisensäurebenzylester und Triäthylamin in THF behandelt.

Beispiel 28

500 mg N-[m-[[$N_5$-(Benzyloxycarbonyl)-$N_2$-t-butoxycarbonyl -L-ornithyl]amino]benzoyl]-β-alaninbenzylester, 10 ml Aethanol, 0,05 ml Essigsäure und 125 mg Pd/C werden 3,5 Stunden unter Wasserstoff gerührt. Das Reaktionsgemisch wird filtriert und das Filtrat im Vakuum eingedampft. Man erhält 333 mg N-[m-[[(S)-$N_2$-(t-Butoxycarbonyl)-L-ornithyl]amino]benzoyl] -β-alaninacetat (1:1), $[\alpha]_D$ = -18,1° (MeOH, C = 0,44%).

Der Ausgangsester, $[\alpha]_D$ = -13,2° (MeOH, c = 0,5%). wird aus $N_2$-(t-Butoxycarbonyl)-$N_5$-(benzyloxycarbonyl)-L-ornithin und N-(m-Aminobenzoyl)-β-alaninbenzylester-trifluoracetat hergestellt.

Beispiel 29

512 mg N-[m-(α-Amino-m-toluamido)benzoyl]-β-alanin (Beispiel 26) und 553 mg 2-[(Aminoiminomethyl)-thio]äthansulfonsäure werden in 3 ml $H_2O$ und 3 ml 1N Natronlauge 4 Tage bei 20 °C gerührt. Der Niederschlag wird abzentrifugiert, 20 Stunden in 5 ml 0,1N Natronlauge gerührt, abzentrifugiert, mit Methanol-Wasser 1:1, Methanol und Aether gewaschen. Man erhält 512 mg N-[m-(α-Guanidino-m-toluamido)benzoyl]-β-alanin, Smp. 314 °C (Zers.).

Beispiel 30

Analog Beispiel 16 wird aus N-[m-[[(S)-$N_2$-(t-Butoxycarbonyl)-L-ornithyl]amino]benzoyl] -β-alaninacetat (1:1) (Beispiel 28) das N-[m-(L-Ornithylamino)benzoyl]-β-alaninformiat (1:1) erhalten. $[\alpha]_D$ = +40,4° (Wasser, c = 0,5%).

Beispiel 31

293 mg N-[5-[[N-(t-Butoxycarbonyl)-β-alanyl]amino]anthraniloyl] -β-alanin-t-butylester werden in 1,3 ml Trifluoressigsäure 3 Stunden bei 20 °C gerührt. Das Lösungsmittel wird im Vakuum eingedampft, der Rückstand in Wasser gelöst und im Vakuum eingedampft. Man erhält 324 mg N-[5-(β-Alanylamino)-anthraniloyl]-β-alanin-trifluoracetat (1:2), MS: 295 (100, M+H).

Zur Herstellung des Ausgangsesters, MS: 468 (18, M+$NH_4$), 451 (100, M+H), werden N-(t-Butoxycarbonyl)-β-alanin und 5-Amino-2-nitrobenzoesäure zur 5-[3-(1-t-Butoxyformamido)propionamido]-2-nitrobenzoesäure, MS: 354 (17, M+H), letztere mit β-Alanin-t-butylester zum N-[5-[3-(1-t-Butoxyformamido)-propionamido]-2-nitrobenzoyl]-β-alanin-t-butylester umgesetzt und dieser an Pd/C in Aethanol hydriert.

Beispiel 32

296 mg N-[m-[2-(α-Azido-p-tolyl)acetamido]benzoyl]-β-alaninbenzylester und 74 mg Pd/C werden in 6 ml Essigsäure 7 Stunden unter Wasserstoff gerührt. Nach Zugabe von 3 ml Wasser wird filtriert und das

Filtrat im Vakuum eingedampft. Der Rückstand wird in Wasser aufgenommen und die Suspension im Vakuum eingedampft. Der Rückstand wird in Methanol verrührt, abgenutscht und getrocknet. Man erhält 125 mg N-[m-[2-(α-Amino-p-tolyl)acetamido]benzoyl]-β-alanin (3:4-Hydrat), Smp. 226 °C.

Der Ausgangsester, Smp. 108-109 °C (aus Aethanol), wird aus (α-Azido-p-tolyl)essigsäure und N-(m-Aminobenzoyl)-β-alaninbenzylester-trifluoracetat hergestellt.

Beispiel 33

A) Eine Lösung von 55 mg N-[N-[m-[[3-(p-Amidinophenyl)-N-(t-butoxycarbonyl) -DL-alanyl]amino]-benzoyl]-3-(t-butoxycarbonyl) -L-alanyl]-3-(1-naphthyl)-L-alanin (1:1 Epimer) in einer Mischung von 10 ml Methylenchlorid und 5 ml Trifluoressigsäure wird unter Begasung mit Argon 2 Stunden bei Raumtemperatur aufbewahrt. Nach Verdampfen der Lösungsmittel erhält man N-[N-[m-[[3-(p-Amidinophenyl)-DL-alanyl]amino]benzoyl] -L-α-aspartyl]-3-(1-naphthyl)-L-alanintrifluoracetat (1:2), Smp. 165 °C (Zers.) (Aethanol/Essigester), Ausbeute: 84% d.Th., MS: 639 $(M+H)^+$.

B) Das Ausgangsmaterial kann auf folgende Weise hergestellt werden:

a) Eine auf 0 °C abgekühlte Lösung von 1,95 g N-Z-Aminobenzoesäure wird unter Rühren durch tropfenweise Zugabe von N-Methylmorpholin auf pH 8 gebracht und hierzu 2 g O-Benzotriazolyl-N,N,N,N-tetramethyluronium-hexafluorphosphat (HBTU) und eine Lösung von 2,1 g H-Asp(OtBu)-OMe in 160 ml DMF addiert. Unter Begasung mit Argon wird 1 Stunde bei 0 °C gerührt und über Nacht im Kühlschrank aufbewahrt. Der nach Verdampfen des Lösungsmittels zurückbleibende Rückstand wird in Essigester aufgenommen und die organische Phase wird mit gesättigter Natriumbicarbonatlösung, Wasser, 10%ige Kaliumhydrogensulfonatlösung und Wasser gewaschen, über Natriumsulfat getrocknet, filtriert und eingedampft. Man erhält 4-t-Butyl-1-methyl-N-[m-[1-(benzyloxy)formamido]benzoyl]-L-aspartat, Smp. 115-116 °C (Aether/Hexan), Ausbeute: 88% d.Th., MS: 457 $(M+H)^+$.

b) Einer Lösung von 27,0 g des Produkts von a) in 200 ml Aceton werden unter Eiskühlung 70 ml 1N NaOH unter Rühren zugetropft und das Rühren wird bei dieser Temperatur 2 Stunden fortgesetzt. Durch Zugabe von 10%iger wässriger Zitronensäure wird das pH auf 4 eingestellt und vom Lösungsmittel befreit. Durch Kristallisation und nachfolgende Extraktion mit Aether erhält man nach dem Umkristallisieren aus Methylenchlorid/Hexan 4-t-Butyl-1-Wasserstoff-N-[m-[1-(benzyloxy)-formamido]benzoyl] -L-aspartat, Smp. 140-142 °C, Ausbeute: 89% d.Th., MS: 443 $(M+H)^+$.

c) In analoger Weise wie unter a) beschrieben erhält man durch Kupplung des Produkts von b) mit Methyl-(S)-α-amino-1-naphthalenpropionat den N-[N-[(m-[1-(Benzyloxy)formamido]benzoyl] -3-(t-butoxycarbonyl)-L-alanyl]-3-(1-naphthyl)-L-alaninmethylester,Smp. 95-98 °C (Essigester/Hexan). Ausbeute: 63% d.Th., MS: 654 $(M+H)^+$.

d) Eine Lösung von 1,9 g des Produkts von c) in 100 ml Methanol wird in Gegenwart von 0,1 g Pd/C 10% hydriert. Nach Aufnahme der theoretischen Menge Wasserstoff wird filtriert und zur Trockene verdampft. Reinigung durch Chromatographie an Kieselgel mit Methylenchlorid/MeOH (98:2) ergibt N-[N-(m-Aminobenzoyl)-3-(t-butoxycartonyl) -L-alanyl]-3-(1-naphthyl)-L-alaninmethylester, Smp. 70-72 °C (Hexan), Ausbeute: 84% d.Th., MS: 520 $(M+H)^+$.

e) Zu einer Lösung von 200 mg N-Boc-3-(p-cyanophenyl)-DL-alanin und 294 mg des Produkts von d) in 10 ml DMF fügt man unter Rühren und Begasung mit Argon 67 mg N-Methylmorpholin und 250 mg HBTU zu und bewahrt die Mischung über Nacht auf. Das nach dem Verdampfen des Lösungsmittel erhältliche Oel wird in Essigester gelöst, die Lösung mit 5% wässriger Natriumbicarbonatlösung und Wasser gewaschen, über $MgSO_4$ getrocknet und eingedampft, der zurückbleibende Schaum durch Chromatographie an Kieselgel mit Essigester gereinigt. Man erhält N-[3-(t-Butoxycarbonyl)-N-[m-[[N-(t-butoxycarbonyl) -3-(p-cyanophenyl)-DL-alanyl]amino]benzoyl]-L-alanyl] -3-(p-naphthyl)-L-alaninmethylester (1:1 Epimere), Smp. 182-185 °C (Essigester/Hexan), Ausbeute: 39% d.Th., MS: 792 $(M+H)^+$.

f) 362 mg des Produkts von e) werden in 40 ml Pyridin und 3 ml Triäthylamin gelöst. Nach Sättigung mit $H_2S$ wird 2 Tage aufbewahrt, in Wasser eingerührt und mit Essigester extrahiert. Das Rohprodukt wird durch Chromatographie an Kieselgel mit Methylenchlorid/Methanol (97:3) gereinigt. Man erhält N-[3-(t-Butoxycarbonyl)-N-[m-[[N-(t-butoxycarbonyl)-3-[p-(thiocarbamoyl)phenyl]-DL-alanyl]amino]-benzoyl]-L-alanyl]-3-(1-naphthyl)-L-alaninmethylester (1:1 Epimere), Smp. 131 °C (Zers.), Ausbeute: 58% d.Th., MS: 826 $(M+H)^+$.

g) Das Thioamid von f) wird in 30 ml Aceton gelöst, mit 0,6 ml Methyljodid versetzt und 3 Stunden unter Rückfluss erhitzt. Nach Filtration und Einengen wird das Produkt durch Zugabe von Aether ausgefällt. Man erhält N-[3-(t-Butoxycarbonyl)-N-[m-[[N-(t-butoxycarbonyl) -3-[p-[(methylthio)-formimidoyl]phenyl] -DL-alanyl]amino]benzoyl]-L-alanyl]-3-(1-naphthyl) -L-alaninmethylester-hydrojodid (1:1 Epimere), Smp. 162-163 °C (Aether), Ausbeute: 75% d.Th., MS: 840 $(M+H)^+$.

h) Eine Lösung von 180 mg Thioimidsäuremethylesterhydrojodid von f) in 30 ml MeOH wird mit 36 mg Ammoniumacetat versetzt und 5 Stunden auf 60 °C erwärmt. Nach dem Abkühlen und Filtrieren wird das Rohprodukt mit Aether ausgefällt. Man erhält N-[N-[m-[[3-(p-Amidinophenyl)-N-(t-butoxycarbonyl) -DL-alanyl]amino]benzoyl]-3-(t-butoxycarbonyl)-L-alanyl]-3-(1-naphthyl)-L-alaninmethylester-hydrojodid (1:1 Epimere), Smp. 170-171 °C (Zers.) (Aether), Ausbeute: 71% d.Th., MS: 809 $(M+H)^+$.
i) Analog Absatz b) erhält man durch alkalische Verseifung des Produkts von h) N-[N-[m-[[3-(p-Amidinophenyl)-N-(t-butoxycarbonyl) -DL-alanyl]amino]benzoyl]-3-(t-butoxycarbonyl) -L-alanyl]-3-(1-naphthyl)-L-alanin (1:1 Epimere), Smp. 263-265 °C (Wasser), Ausbeute: 94% d.Th., MS: 795 $(M+H)^+$.

Beispiel 34

Analog Beispiel 33 durch Acidolyse von N-[N-[m-(p-Amidinobenzamido)benzoyl]-3-(t-butoxycarbonyl) -L-alanyl]-3-(1-naphthyl)-L-alanin erhält man N-[N-[m-(p-Amidinobenzamido)benzoyl]-L-α-aspartyl] -3-(1-naphthyl)-L-alanintrifluoracetat (1:1,9), Smp. 130 °C (Zers.) (Hexan), Ausbeute: 54% d.Th., MS: 596 $(M+H)^+$.

Das Ausgangsmaterial kann wie folgt hergestellt werden:

a) Durch Kupplung von 4-Cyanobenzoesäure mit N-[N-(m-Aminobenzoyl)-3-(t-butoxycarbonyl)-L-alanyl] -3-(1-naphthyl)-L-alaninmethylester erhält man den N-[3-(t-Butoxycarbonyl)-N-[m-(p-cyanobenzamido)-benzoyl] -L-alanyl]-3-(-1-naphthyl)-L-alaninmethylester, Ausbeute: 56% d.Th., MS: 649 $(M+H)^+$.
b) Durch Thionierung, Methylierung und Ammonolyse des Produkts von a) erhält man das N-[N-[m-(p-Amidinobenzamido)benzoyl]-3-(t-butoxycarbonyl) -L-alanyl]-3-(-1-naphthyl)-L-alaninmethylester-hydrojodid, Smp 87 °C (Zers.) (Hexan), Ausbeute: 74% d.Th., MS: 666 $(M+H)^+$.
c) Durch alkalische Verseifung der Vorstufe erhält man N-[N-[m-(p-Amidinobenzamido)benzoyl]-3-(t-butoxycarbonyl)-L-alanyl]-3-(-1-naphthyl)-L-alanin, Smp. 230 °C (Zers.), Ausbeute: 34% d.Th., MS: 652 $(M+H)^+$.

Beispiel 35

Analog Beispiel 32 erhält man aus 579 mg N-[m-[p-[N-(Benzyloxycarbonyl)amidino]benzamido]-benzoyl] -β-alaninbenzylester 268 mg N-[m-(p-Amidinobenzamido)benzoyl-β-alanin, Smp. 276 °C (Zers.).

Zur Herstellung des Ausgangsesters (Smp. 192 °C) werden p-Amidinobenzoylchlorid-hydrochlorid, m-Aminobenzoyl-β-alaninbenzylestertrifluoracetat und Triäthylamin in THF 20 Stunden bei 0-5 °C reagieren gelassen. Anschliessend wird mit Chlorameisensäurebenzylester und Triäthylamin versetzt und 30 Stunden bei 0-5 °C reagieren gelassen.

Beispiel 36

Analog Beispiel 1 erhält man aus N-[m-[[N-[N,N′ -Bis(t-butoxycarbonyl)amidino]glycyl]amino]benzoyl] -β-alaninbenzylester das N-[m-[(N-Amidinoglycyl)amino]benzoyl]-β-alaninformiat(2:1), MS: 308 (33, M+H).

Den Ausgangsester [MS: 598 (4, M+H)] erhält man aus Glycin und N,N′-Bis(t-butoxycarbonyl)-S-methylisothioharnstoff über N-[N,N′-Bis(t-butoxycarbonyl)amidino]glycin und N-(m-Aminbenzoyl)-β-alaninbenzylestertrifluoracetat.

Beispiel 37

Analog Beispiel 1 erhält man aus N-[m-[$N_6$-[(Benzyloxy)carbonyl]-$N_2$-(tert-butoxycarbonyl) -L-lysyl]-benzoyl]-β-alaninbenzylester das N-[m-(L-Lysylamino)benzoyl]-β-alaninfotmiat (2:3), MS: 337 (7, M+H).

Das Ausgangsmaterial [MS: 661 (7, M+H)] erhält man aus $N_6$-(Benzyloxycarbonyl)-$N_2$-(tert.-butoxycarbonyl)-L-lysin-h ydroxysuccinimidester und N-(m-Aminobenzoyl)-β-alaninbenzylester in siedendem THF.

Beispiel 38

Analog Beispiel 1 erhält man aus DL-N-[m-[5-(1-tert.-Butoxyformamido)valeramido]benzoyl] -3-phenyl-β-alaninbenzylester das DL-N-[m-(5-Aminovaleramido)-benzoyl]-3-phenyl-β-alanin-formiat(2:1), MS: 384 (100, M+H).

Das Ausgangsmaterial kann wie folgt hergestellt werden:

a) DL-3-Amino-3-phenylpropionsäure wird mit Benzylalkohol und p-Toluolsulfonsäure zu DL-3-Amino-3-phenylpropionsäurebenzylester-p-toluolsulfonat verestert, MS: 164 (23, M-$C_7H_7$).

b) 5-[1-(tert.-Butoxy)formamidovaleriansäure wird mit m-Aminobenzoesäure zu m-[5-(1-tert.-Butoxyformamido)valeramido]-benzoesäure, Smp. 188 ° C gekoppelt und letzteres mit 3-Amino-3-phenylpropionsäurebenzylester-p-toluolsulfonat zu DL-N-[m-[5-(1-tert.Butoxyformamido)valeramido]-benzoyl]-3-phenyl-β-alaninbenzylester umgesetzt, MS: 574 (17, M + H).

Beispiel 39

Analog Beispiel 16 erhält man aus N-[3-[5-(1-tert.-Butoxyformamido)valeramido]-4-chlorbenzoyl] -β-alanin-tert.-butylester das N-[3-(5-Aminovaleramido)-4-chlorbenzoyl -β-alanin-formiat (2:1), Smp. 184-186 ° C.

Zur Herstellung des Ausgangsmaterials werden 5-[1-(tert.-Butoxy)formamidovaleriansäure und 3-Amino-4-chlorbenzoesäure zur 3-[5-(1-tert.-Butoxyformamido)valeramido]-4-chlorbenzoesäure, Smp. 203 ° C, letztere mit β-Alanin-tert.-butylester zum N-[3-[5-(1-tert.-Butoxyformamido)valeramido]-4-chlorbenzoyl] -β-alanin-tert.-butylester gekoppelt, MS: 498 (60, M + H).

Beispiel 40

Analog Beispiel 1 erhält man aus N-[3-[5-(1-tert.-Butoxyformamido)valeramido]-p-toluoyl] -β-alaninbenzylester das N-[3-(5-Aminovaleramido)-p-toluoyl] -β-alanin, Smp. 212-213 ° C, als Hydrat (2:1).

Zur Herstellung des Ausgangsmaterials werden 5-[1-(tert.-Butoxyformamido)valeriansäure und 3-Amino-p-toluoylsäure zur 3-[5-(1-tert.-Butoxyformamido)valeramido]-p-toluoylsäure, Smp. 212 ° C, letztere mit β-Alaninbenzylester zum N-[3-[5-(1-tert.-Butoxyformamido)valeramido]-p-toluoyl] -β-alaninbenzylester gekoppelt, Smp. 112-113 ° C.

Beispiel 41

Analog Beispiel 31 erhält man aus DL-N-[-m-[5-(1-tert.-Butoxyformamido)valeramido]-benzoyl] -3-(5-indanyl)-β-alanin das DL-N-[m-(5-Aminovaleramido)benzoyl] -3-(5-indanyl)-β-alanin-trifluoracetat (2:3), MS: 424 (36, M + H).

Das Ausgangsmaterial, MS: 524 (87, M + H) erhält man aus m-[5-(1-tert.-Butoxyformamido)valeramido]-benzoesäure und β-Amino-5-indanpropionsäure.

Beispiel 42

Analog Beispiel 5 erhält man aus N-[m-[trans-4-[[1-(Benzyloxy)formamido]-methyl] cyclohexancarboxamido]benzoyl]-β-alaninbenzylester das N-[m-[trans-4-(Aminomethyl)cyclohexancarboxamido]benzoyl] -β-alanin, Smp. 248-249 ° C.

Das Ausgangsmaterial, Smp. 145-146 ° C, erhält man durch Kopplung vom trans-4-[[1-(Benzyloxy)-formamido]methyl]-cyclohexancarbonsäure mit N-(m-Aminobenzoyl)-β-alaninbenzylester.

Beispiel 43

Analog Beispiel 5 erhält man aus N-[m-[m-[N-[(Benzyloxy)carbonyl]amidino]benzamido]benzoyl] -β-alaninbenzylester das N-[m-(m-Amidinobenzamido)benzoyl -β-alanin, Smp. 221-223 ° C (Zersetzung).

Zur Herstellung des Ausgangsmaterials, Smp. 182-183 ° C werden 3-Cyanobenzoesäure und N-(m-Aminobenzoyl)-β-alaninbenzylester-trifluoracetat zum N-[m-(m-Cyanobenzamido)benzoyl] -β-alaninbenzylester gekoppelt, MS: 428 (100, M + H), und dieser mit Schwefelwasserstoff und Triäthylamin in Pyridin in den N-[m-[m-(Thiocarbamoyl)-benzamido]benzoyl]-β-alaninbenzylester, Smp. 128-131 ° C übergeführt. Letzterer wird dann mit Methyljodid in Aceton und anschliessend mit Ammoniumacetat in Methanol umgesetzt und mit Chlorameisensäurebenzylester und Triäthylamin in THF behandelt.

Beispiel 44

Analog Beispiel 16 erhält man aus DL-N-[m-[5-[(E/Z)-2,3-Bis(tert.-butoxycarbonyl)-guanidino] valeramido]benzoyl]-3-(5-indanyl)-β-alanin das DL-N-[m-(5-Guanidinovaleramido)-benzoyl] -3-(5-indanyl)-β-alanin-formiat (2:1), MS: 466 (100, M + H).

Das Ausgangsmaterial wird aus DL-N-[m-(5-Aminovaleramido)benzoyl]-3-(5-indanyl) -β-alanin-trifluoracetat und N-N′-Bis(tert.-butoxycarbonyl) -S-methyl-isothioharnstoff erhalten.

Beispiel 45

Analog Beispiel 5 erhält man durch Hydrierung in Essigsäure-Methanol 1:1 von N-[[m-[α[1-(Benzyloxy)-formamido]-p-toluamido]phenyl]acetyl] -β-alanin das N-[[m-(α-Amino-p-toluamido)phenyl]acetyl]-β-alanin, MS: 356 (100, M + H).

Das Ausgangsmaterial, Smp. 208-210 ° C, kann hergestellt werden, indem man

a) m-Nitrophenylessigsäure und β-Alaninbenzylester zu N-[(m-Nitrophenyl)acetyl]-β-alaninbenzylester koppelt, MS: 236 (6, M-$C_6H_5$-$CH_2O$).

b) diesen zu N-[(m-Aminophenyl)acetyl]-β-alanin hydriert, MS: 223 (100, M + H) und

c) letzteres mit α-[1-(Benzyloxy)formamido] -p-toluylsäure koppelt.

Beispiel 46

Analog Beispiel 16 erhält man aus N-[2-(Benzyloxy)-5-[5-(1-tert.-butoxyformamido)valeramido] benzoyl]-β-alanin in 2 Stunden das N-[5-(5-Aminovaleramido)-2-(benzyloxy)-benzoyl-β-alanin, Smp. 227-228 ° C.

Das Ausgangsmaterial kann wie folgt hergestellt werden:

a) 5-[1-(tert.-Butoxy)formamidovaleriansäure wird mit 5-Aminosalicylsäure zu 5-[5-(1-tert.-Butoxyformamido) valeramido]-salicylsäure, Smp. 181 ° C, gekoppelt.

b) Daraus erhält man mit Benzylbromid und Kaliumcarbonat in DMF tert.-Butyl[4-[[4-(benzyloxy)-3-[-(benzyloxy)carbonyl]phenyl] -carbamoyl]butyl]carbamat, Smp. 104-106 ° C.

c) Durch Verseifung mit NaOH in t-Butanol erhält man daraus die 2-(Benzyloxy)-5-[5-(1-tert.-butoxyformamido)valeramido]-benzoesäure, Smp. 133-134 ° C.

d) Diese wird mit β-Alaninbenzylester zu N-[2-(Benzyloxy)-5-[5-(1-tert.-butoxyformamido)valeramido] benzoyl]-β-alaninbenzylester, Smp. 97-99 ° C, gekoppelt.

e) Letzterer wird in Methanol mit NaOH verseift zu N-[2-(Benzyloxy)-5-[5-(1-tert.-butoxyformamido)-valeramido] benzoyl]-β-alanin, MS: 514 (51, M ° H).

Beispiel 47

Analog Beispiel 16 erhält man aus N-[2-(Benzyloxy)-5-[5-[(E/Z)-N,N′-bis(tert.-butoxycarbonyl) guanidino]valeramido]benzoyl]-β-alanin das N-[2-(Benzyloxy)-5-(5-guanidinovaleramido)-benzoyl] -β-alanin, Smp. 242-245 ° C.

Das Ausgangsmaterial, MS: 656 (32, M + H), erhält man aus N-[5-(5-Aminovaleramido)-2-(benzyloxy)-benzoyl] -β-alanin, (Beispiel 46), und N,N′-Bis(tert.-butoxycarbonyl)-S-methylisothioharnstoff.

Beispiel 48

Analog Beispiel 16 erhält man aus N-[5-[5-[(E oder Z)-2,3-Bis(tert.-butoxycarbonyl)-guanidino]-valeramido] salicyloyl]-β-alanin das N-[5-(5-Guanidinovaleramido)salicyloyl]-β-alanin, Smp. >260 ° C, MS: 366 (100, M + H).

Das Ausgangsmaterial, Smp. >250 ° C, MS: 566 (28, M + H), erhält man aus N-[2-(Benzyloxy)-5-[5-[-(E/Z)-N,N′-bis-(tert.-butoxycarbonyl) guanidino]valeramido]-benzoyl]-β-alanin in Aethanol mit Wasserstoff und Pd/C.

Beispiel 49

Analog Beispiel 5 erhält man aus N-[α-[p-[N-[(Benzyloxy)carbonyl]amidino]benzamido] -m-toluoyl]-β-alaninbenzylester das N-[α-(p-Amidinobenzamido)-m-toluoyl]-β-alanin, Smp 286 ° C.

Das Ausgangsmaterial, Smp 157 ° C, kann wie folgt hergestellt werden:

a) α-(1-tert.-Butoxyformamido)-m-toluoylsäure und β-Alaninbenzylester werden zu N-[α-(1-tert.-Butoxyformamido)-m-toluoyl] -β-alaninbenzylester gekoppelt, MS: 413 (4, M + H).

b) Daraus erhält man mit Trifluoressigsäure das N-[(α-Amino)-m-toluoyl]-β-alanin-benzylester-trifluoracetat.

c) Letzteres wird zunächst in Methylenchlorid in Gegenwart von gesättigter wässeriger Natriumhydrogencarbonatlösung mit p-Amidinobenzoylchlorid und anschliessend in Gegenwart von Natriumcarbonatlösung mit Chlorameisensäurebenzylester ungesetzt.

Beispiel 50

Analog Beispiel 1 erhält man aus N-[α-[p-[N-[(Benzyloxy)carbonyl]amidino]benzamido] -p-toluoyl]-β-alaninbenzylester das N[α-(p-Amidinobenzamido)-p-toluoyl]-β-alanin, Smp. >300°, MS: 369 (100, M+H).

Das Ausgangsmaterial, Smp. 188-192°C, kann wie folgt hergestellt werden:

a) α-(1-tert.-Butoxyformamido)-p-toluoylsäure und β-Alaninbenzylester werden zu N-[α-(1-tert.-Butoxyformamido)-p-toluoyl] -β-alaninbenzylester, Smp.113-116°C, gekoppelt.

b) Daraus erhält man mit Trfluoressigsäure das N-[(α-Amino)-p-toluoyl]-β-alaninbenzylester-trifluoracetat.

c) Letzteres wird zunächst mit p-Amidino-benzoylchlorid und anschliessend mit Chlorameisensäurebenzylester umgesetzt.

Beispiel 51

550 mg N-[p-[p-[N,N,N′-Tris(tert.-butoxycarbonyl)-amidino]benzamido]benzoyl]-β-alanin wurden 1 Stunde in 10 ml Trifluoressigsäure gerührt. Das Lösungsmittel wurde im Vakuum eingedampft, der Rückstand in Wasser gelöst und die Lösung wieder eingedampft. Der verbliebene feste Rückstand wurde in Wasser aufgenommen und die Suspension mit Ammoniak auf pH 8-9 eingestellt. Nach kurzem Rühren wurde das kristalline N-[p-(p-Amidinobenzamido)benzoyl] -β-alanin abgenutscht, mit Wasser gewaschen und getrocknet, Smp. >250°, MS: 355 (21, M+H).

Das Ausgangsmaterial kann wie folgt hergestellt werden:

a) p-(Amidinobenzoesäuremethylester-hydrochlorid wird in Methylenchlorid und wässerige Natriumcarbonatlösung mit Di-tert.-butyldicarbonat zu p-[N-(tert.-Butoxycarbonyl)amidino]benzoesäuremethylester umgesetzt, Smp. 157°C.

b) Daraus erhält man mit Di-tert.-butyldicarbonat und p-Dimethylaminopyridin in Acetonitril den p-[(E/Z)-Tri(tert.-butoxycarbonyl)-amidino] benzoesäuremethylester, Smp. 70-73°C.

c) Dieser wird mit methanolischer Natronlauge zu p-[(E/Z)-Tri(tert.-butoxycarbonyl)amidino] benzoesäure, Smp. 157°C, verseift.

d) N-(p-Aminobenzamido)-β-alanin wird mit Benzylalkohol und p-Toluolsulfonsäure zu N-(p-Aminobenzamido)-β-alaninbenzylester umgesetzt, Smp. 96-97°C.

e) Letzterer wird mit p-[(E/Z)-Tri(tert.-butoxycarbonyl)amidino]benzoesäure zu N-[p-[p-[N,N,N′-Tris(tert.-butoxycarbonyl)amidino] benzamido]benzoyl]-β-alaninbenzylester gekoppelt, Smp. 164-165°C.

f) Durch katalytische Hydrierung erhält man daraus das N-[p-[p-[N,N,N′-Tris(tert.-butoxycarbonyl)amidino] benzamido]benzoyl]-β-alanin, Smp. >160°, (Zers.).

Beispiel 52

Analog Beispiel 5 erhält man nach 48 Stunden aus N-[m-[2-[p-[[1-(Benzyloxy)formamido]formimidoyl]-phenyl] acetamido]benzoyl]-β-alaninbenzylester das N-[m-[2-(p-Amidinophenyl)acetamido]benzoyl]-β-alanin, Smp. 270°C (Zers.), als Hydrat (1:1).

Das Ausgangsmaterial, Smp. 167°C, kann wie folgt hergestellt werden:

a) p-Cyanophenylessigsäure und N-(m-Aminobenzoyl) -β-alaninbenzylester-trifluoracetat werden zu N-[m-[2-(p-Cyanophenyl)acetamido]benzoyl] -β-alaninbenzylester gekoppelt, Smp. 98-99°C.

b) Dieser wird mit Schwefelwasserstoff und Triäthylamin in Pyridin in den N-[m-[2-[p-(Thiocarbamoyl)-phenyl]acetamido] benzoyl]-β-alaninbenzylester übergeführt, Smp. 163°C (Zers.).

c) Letzteres wird mit Methyljodid in Aceton, anschliessend mit Ammoniumacetat in Methanol und schliesslich mit Chlorameisensäurebenzylester in Essigester und gesättigter Natriumcarbonatlösung umgesetzt.

Beispiel 53

Analog Beispiel 5 erhält man aus N-[m-[3-[1-[(Benzyloxy)carbonyl] -4-piperidinyl]-propionamido]-benzoyl] -β-alaninbenzylester das N-[m-[3-(4-Piperidinyl)propionamido]benzoyl] -β-alanin, Smp. 163°C (MeOH), als Solvat mit MeOH (4:3).

Das Ausgangsmaterial, MS: 572 (11, M+H), erhält man durch Kopplung von 1-[(Benzyloxy)carbonyl] -4-piperidinpropionsäure mit N-(m-Aminobenzoyl) -β-alaninbenzylester-trifluoracetat.

Beispiel 54

Analog Beispiel 16 erhält man in 4 Stunden aus N-[2-(Benzyloxy)-5-[p-[N-(tert.-butoxycarbonyl)-amidino] benzamido]benzoyl]-β-alanin das N-[5-(p-Amidinobenzamido)-2-(benzyloxy)-benzoyl]-β-alanin, Smp. 215-217° (lyophilisiert), als Hydrat (1:4).

Das Ausgangsmaterial, Smp. >235°C, (Zers.), kann wie folgt hergestellt werden:

a) 5-Aminosalicylsäure wird in tert.-Butanol und Wasser mit Di-tert.-butyldicarbonat und Triäthylamin zu 5-(1-tert.-Butoxyformamido)salicylsäure, Smp. >270°, MS: 253 (8, M), umgesetzt.

b) Daraus erhält man mit Benzylbromid und Kaliumcarbonat in Aceton den 2-(Benzyloxy) -5-(1-tert.-butoxyformamido)benzoesäurebenzylester, Smp. 113-115°C.

c) Dieser wird in methanolischer Natronlauge zur 2-(Benzyloxy) -5-(1-tert.-butoxyformamido)benzoesäure verseift, Smp. 136-139°C.

d) Koppelt man letztere mit β-Alaninmethylester so erhält man N-[2-(Benzyloxy)-5-(1-tert.-butoxyformamido)benzoyl]-β-alaninmethylester, Smp. 139-141°C.

e) In Trifluoressigsäure erhält man daraus den N-[5-Amino-2-(benzyloxy)benzoyl] -β-alaninmethylester, Smp. 80-83°C.

f) Letzterer wird mit p-Amidinobenzoylchlorid und anschliessend mit Di-tert.-butyldicarbonat zum N-[2-(Benzyloxy)-5-[p-[N-(tert.-butoxycarbonyl)amidino] benzamido]benzoyl]-β-alaninmethylester umgesetzt, Smp. >270° (Zers.), MS: 575 (14, M+H).

g) Verseifung dieses Esters mit Natronlauge in Methanol-THF gibt das Ausgangsmaterial.

Beispiel 55

173 mg N-[4-(Benzyloxy)-3-[p-[N-(tert.-butoxycarbonyl)amidino] benzamido]benzoyl]-β-alanin werden in 4 ml Ameisensäure 4 Stunden bei 20° gerührt. Das Reaktionsgemisch wird im Vakuum eingedampft, der Rückstand in Wasser suspendiert und mit Ammoniak auf pH 8-9 eingestellt. Nach kurzem Rühren wird abgenutscht, mit Wasser gewaschen und bei 50° im Vakuum getrocknet. Man erhält 136 mg N-[3-(p-Amidinobenzamido)-4-(benzyloxy)benzoyl] -β-alanin, Smp. >250° MS: 461 (41, M+H), als Hydrat (1:1).

Das Ausgangsmaterial, Smp. >190° (Zers.), MS: 561 (29, M+H), kann in Analogie zu der in Beispiel 54 beschriebenen Weise aus 3-Amino-4-hydroxybenzoesäure über folgende Zwischenprodukte hergestellt werden:

a) 3-(1-tert.-Butoxyformamido)-4-hydroxybenzoesäure, Smp. >185° (Zers.), MS: 197 (40, M-$C_4H_8$).

b) 4-(Benzyloxy)-3-(1-tert.-butoxyformamido) benzoesäurebenzylester, MS: 433 (3, M).

c) 4-(Benzyloxy)-3-(1-tert.-butoxyformamido)-benzoesäure, Smp. 200-201°C.

d) N-[4-(Benzyloxy)-3-(1-tert.-butoxyformamido)benzoyl]-β-alaninmethylester, MS: 429 (58, M+H).

e) N-[3-Amino-4-(benzyloxy)benzoyl] -β-alaninmethylester, Smp. 121-122°C.

f) N-[4-(Benzyloxy)-3-[p-[N-(tert.-butoxycarbonyl)amidino] benzamido]benzoyl-β-alaninmethylester, Smp. >260°C (Zers.), MS: 575 (29, M+H).

Beispiel 56

Analog Beispiel 55 erhält man aus N-[3-[p-[N-(tert.-Butoxycarbonyl)amidino]benzamido] -4-hydroxybenzoyl]-β-alanin das N-[3-(p-Amidinobenzamido) -4-hydroxybenzoyl-β-alanin, Smp. 253-255 (Zers.).

Das Ausgangsmaterial erhält man aus N-[4-(Benzyloxy)-3-[p-[N-(tert.-butoxycarbonyl)-amidino] benzamido]benzoyl-β-alaninmethylester durch katal. Hydrierung an Pd/C in Aethanol-DMF 2:1 zu N-[3-[p-[N-(tert.-Butoxycarbon)amidino]benzamido] -4-hydroxybenzoyl]-β-alaninmethylester, Smp. >180° (Zers.), MS: 485 (78, M+H) und Verseifung des letztgenannten in methanolischer Natronlauge.

Beispiel 57

Analog Beispiel 55 erhält man aus N-[5-[p-[N-(tert.-Butoxycarbonyl)amidino]benzamido] salicyloyl]-β-alanin das N-[5-(p-Amidinobenzamido)salicyloyl] -β-alanin, Smp. >260°, MS: 371 (8, M+H).

Das Ausgangsmaterial, Smp. 189-195 (Zers.), erhält man aus N-[2-(Benzyloxy)-5-[p-[N-(tert.-butoxycarbonyl) amidino]benzamido]benzoyl]-β-alaninmethylester durch katal. Hydrierung an Pd/C in DMF zu N-[5-[p-[N-(tert.-Butoxycarbonyl)amidino] benzamido]salicyloyl]-β-alaninmethylester, Smp. 177-179°C und Verseifung des letzteren in methanolischer Natronlauge.

Beispiel 58

Analog Beispiel 31 erhält man aus N-[m-[3-[1-[(E oder Z)-N,N'-Bis(tert.-butoxycarbonyl)amidino] -4-piperidinyl]propionamido]benzoyl]-β-alanin das N-[m-[3-(1 Amidino-4-piperidinyl)propionamido]benzoyl] -β-alanin-trifluoracetat (2:3), MS: 390 (38, M + H).

Das Ausgangsmaterial, Smp. >140 °C (Zers.), erhält man aus N-[m-[3-(4-Piperidinyl)-propionamido]-benzoyl] -β-alanin und N,N'-Bis(tert.-butoxycarbonyl)-S-methylisothioharnstoff.

Beispiel 59

Eine Lösung von 3-(tert.-Butoxycarbonyl)-N-[m-[p-[-1-(methylthio)-formimidoyl] benzamido]benzoyl]-L-alaninmethylester-hydrojodid in Methanol wird in analoger Weise wie in Beispiel 33 B) h) beschrieben mit Ammoniumacetat umgesetzt. Man erhält in 71% Ausbeute N-[m-(p Amidinobenzamido)benzoyl] -3-(tert.-butoxycarbonyl)-L-alaninmethylester-hydrojodid in Form eines farblosen Festkörpers, Smp. 118-120 °C (Aether/Isopropyläther), MS: 469 $(M + 1)^+$.

Das Ausgangsmaterial kann wie folgt hergestellt werden:

a) 4-Cyanobenzoesäure wird analog Beispiel 1 mit 2-Chlor-4,6-dimethoxy-1,3,5-triazin und N-Methylmorpholin aktiviert und anschliessend mit 3-Aminobenzoesäure in $DMF/CH_2Cl_2$ umgesetzt. Man erhält m-(p-Cyanobenzamido)benzoesäure in Form farbloser Kristalle. Ausbeute = 70%, Smp. 267 °C (Essigester/Acetonitril).

b) m-(p-Cyanobenzamido)benzoesäure wird in gleicher Weide wie unter a) beschrieben aktiviert und mit dem p-Toluolsulfonat von H-Asp(OtBu)OMe in DMF bei Raumtemperatur gekoppelt. Man isoliert 3-(tert.-Butoxycarbonyl)-N-[m-(p-Cyanobenzamido) benzoyl]-L-alanin-methylester, Ausbeute: 65%, Smp. 83-84 °C (Hexan).

c) Das Nitrat von b) wird in analoger Weide wie in Beispiel 33 B) f) beschrieben mit $H_2S$ behandelt, wobei 3-(tert.-Butoxycarbonyl)-N-[m-(p-(thiocarbamoyl)benzamido] benzoyl]-L-alaninmethylester gewonnen wird. Ausbeute: 93%, Smp. 97-99 °C (Hexan).

d) Die Umsetzung des Thioamids von c) mit Methyljodid erfolgt analog Beispiel 33 B) g). Man erhält 3-(t-Butoxycarbonyl)-N-[m-[p-[1-(methylthio)formimidoyl] benzamido]benzoyl]-L-alaninmethylester-hydrojodid. Ausbeute: 90%, Smp. 167-169 °C (Zers., Aether), MS: 500 $(M + 1)^+$.

Beispiel 60

Eine Lösung von N-[m-(p-Amidinobenzamido)benzoyl]-3-(t-butoxycarbonyl)-L-alaninmethylesterhydrojodid in $CH_2Cl_2$/Trifluoressigsäure wird 3 Stunden bei Raumtemperatur stehen gelassen. Nach Entfernen der Lösungsmittel und Umkristallisation des Rückstandes aus Aethanol/Aether erhält man (S)-3-[m-(p-Amidinobenzamido)benzamido]-3-(methoxycarbonyl)propionsäure-trifluoracetat. Ausbeute: 75%, Smp. 132-134 °C (Zersetzung), MS: 413 $(M + H)^+$.

Beispiel 61

Eine Lösung von N-[m-(p-Amidinobenzamido)benzoyl]-3-(t-butoxycarbonyl)-L-alaninmethylesterhydrojodid in Methanol wird mit 1N Natronlauge behandelt. Nach 3 Stunden bei Raumtemperatur neutralisiert man mit 1N Salzsäure. Das ausgefallene Produkt, N-[m-(p-Amidinobenzamido)benzoyl]-3-(tert.-butoxycarbonyl)-L-alanin wird abgenutscht und getrocknet. Ausbeute: 75%, Smp. 218-220 °C, MS: 455 $(M + H)^+$.

Beispiel 62

Aus N-[m-(p-Amidinobenzamido)benzoyl] -3-(tert.-butoxycarbonyl) -L-alanin erhält man in analoger Weise wie in Beispiel 60 N-[m-(p-Amidinobenzamido)benzoyl] -L-asparagintrifluoracetat (1:2). Ausbeute: 80%, Smp. 107-108 °C ($Et_2O$; Zers.), MS: 399 $(M + 1)^+$.

Beispiel 63

Die Ammonolyse von (S)-3-[m-(p-Amidinobenzamido)benzamido] -3-(methoxycarbonyl)propionsäure in $NH_3/CH_3OH$ ergibt nach Entfernung der Lösungsmittel (S)-3-[m-(p-Amidinobenzamido)benzamido] -3-(aminocarbonyl)propionsäure. Ausbeute: 50%, Smp. 248-249 °C (MeOH, Zers.).

Beispiel 64

Die Kupplung von N-[N-(m-Aminobenzoyl)-3-tert.-butoxycarbonyl -L-alanyl]-3-phenyl-L-alanin-tert.-butylester mit 1-Amidino-4-piperidincarbonsäure erfolgt analog Beispiel 33 B) a) in Gegenwart von Pyridiniumhydrochlorid. Das nach üblicher Aufarbeitung erhaltene Produkt wird sogleich mit Trifluoressigsäure/$CH_2Cl_2$ behandelt, wobei man nach Abdampfen der Lösungsmittel und Umkristallisation aus Methanol/Essigester N-[N-[m-(1-Amidino-4-piperidincarboxamid)benzoyl] -L-α-aspartyl]-3-phenyl-L-alanin-trifluoracetat erhalten wird. Ausbeute: 11%, Smp. 150 °C, MS: 553 $(M+H)^+$.

Das Ausgangsmaterial kann wie folgt hergestellt werden:

Durch Kupplung von 3-Aminobenzoesäure mit H-Asp(OtBu)-Phe-OtBu (gewonnen aus der Kondensation von Z-Asp(OtBu)-OH mit H-Phe-OtBu gefolgt von Hydrogenolyse) auf die in Beispiel 59 a) beschriebene Art erhält man N-[N-(m-Aminobenzoyl) -3-t-butoxycarbonyl-L-alanyl] -3-phenyl-L-alanin-t-butylester. MS: 512 $(M+1)^+$.

Beispiel 65

Analog Beispiel 60 erhält man durch Acidolyse von N-[N-[m-[2-(p-Amidinophenyl)acetamido]benzoyl] -3-(tert.-butoxycarbonyl)-L-alanyl]-3-phenyl -L-alanin-tert.-butylesterhydrojodid (1:1). das Trifluoracetatsalz von N-[N-[m-[2-(p-Amidinophenyl)acetamido]benzoyl] -L-α-aspartyl]-3-phenyl-L-alanin. Ausbeute: 51%, Smp. 160 °C (Essigester/Hexan, Zers.), MS: 560 $(M+1)^+$.

Das Ausgangsmaterial kann wie folgt hergestellt werden:

a) Kupplung von N-[N-(m-Aminobenzoyl) -3-t-butoxycarbonyl-L-alanyl]-3phenyl-L-alanin-t-butylester (Beispiel 64) mit 4-Cyanophenylessigsäure analog Beispiel 33 B) a) ergibt N-[3-(tert.-Butoxycarbonyl)-N-[m-[2-(p-cyanophenyl)acetamido] benzoyl]-L-alanyl]-3-phenyl-L-alanin-tert.-butylester. Ausbeute: 64%, Smp. 90 °C (Essigester/Hexan), MS: 655 $(M+1)^+$.

b) Durch Thionierung des Produktes von a) gemäss Beispiel 33 B) f) erhält man N-[3-(tert.-Butoxycarbonyl)-N-[m-[2-[p-(thiocarbamoyl) phenyl]acetamido]benzoyl]-L-alanyl]-3-phenyl-L-alanin-tert.-butylester. Ausbeute: 78%, Smp. 99 °C (Essigester/Hexan, Zers.), MS: 689 $(M+1)^+$.

c) Durch Methylierung des Produktes von b) analog Beispiel 33 B) g) gewinnt man N-[3-(tert.-Butoxycarbonyl)-N-[m-[2-[p-[1-(methylthio) formimidoyl]phenyl]acetamido]benzoyl-L-alanyl]-3-phenyl-L-alanin-tert.-butylesterhydrojodid. Ausbeute: 77%, Smp. 115 °C (Essigester/Hexan, Zers.), MS: 703 $(M+1)^+$.

d) Ammonolyse des Produktes von c) analog Beispiel 33 B) h) ergibt N-[N-[m-[2-(p-Amidinophenyl)-acetamido]benzoyl]-3-(tert.-butoxycarbonyl)-L-alanyl] -3-phenyl-L-alanin-tert.-butylesterhydrojodid. Ausbeute: 89%, Smp. 125 °C (Essigester/Hexan, Zers.), MS: 672 $(M+1)^+$.

Beispiel A

Eine Verbindung der Formel I kann man in an sich bekannter Weise als Wirkstoff zur Herstellung von Tabletten folgender Zusammensetzung verwenden:

| | pro Tablette |
|---|---|
| Wirkstoff | 200 mg |
| mikrokristalline Cellulose | 155 mg |
| Maisstärke | 25 mg |
| Talk | 25 mg |
| Hydroxypropylmethylcellulose | 20 mg |
| | 425 mg |

Beispiel B

Eine Verbindung der Formel I kann man in an sich bekannter Weise als Wirkstoff zur Herstellung von Kapseln folgender Zusammensetzung verwenden:

| | pro Kapsel |
|---|---|
| Wirkstoff | 100,0 mg |
| Maisstärke | 20,0 mg |
| Milchzucker | 95,0 mg |
| Talk | 4,5 mg |
| Magnesiumstearat | 0,5 mg |
| | 220,0 mg |

**Patentansprüche**
**Patentansprüche für folgende Vertragsstaaten : AT, BE, CH, DE, FR, GB, IT, LI, LU, NL, SE**

**1.** Verbindungen der Formel

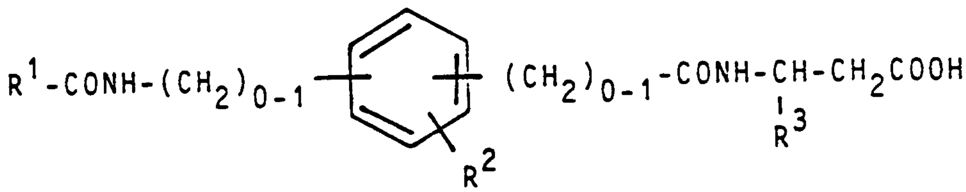
I

worin $R^1$ eine Gruppe der Formel

$-C(H,R^a)-CH_2)_nNH-R^b$ (R-1)

$-(T)_{1\ oder\ 0}-C_6H_4-CH_2NH-R^c$ (R-2)

$-(T)_{1\ oder\ 0}-C_6H_4-(NH)_{1\ oder\ 0}-C(NH)NH_2$ (R-3)

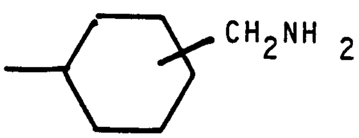
R-4 oder
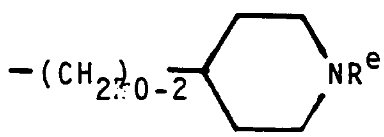
R-5

$R^a$ H, $NH_2$, $-NHCOO-C_{1-4}$-Alkyl, -NH-Z oder $-NHCOCH_2N(Y)-CH_2CH_2NH-Y$, und n 1-6 oder, falls $R^a$ H ist, n auch 0 sein kann,
Y H, Boc oder Z;
$R^b$ H, Amidino oder $-C(NH)-(CH_2)_{0-3}-CH_3$;
$R^c$ H oder Amidino;
T $-CH_2-$, $-CH=CH-$ oder $-C(H,R^d)-CH_2-$ ist;
$R^d$ die gleiche Bedeutung wie $R^a$ hat oder -NHCO-phenyl, -NHCO-phenylen-$N_3$ oder $-NHSO_2$-Aryl ist;
$R^e$ H oder Amidino;
$R^3$ H, $-CONH_2$, $-COR^f$, $COOR^g$ oder Aryl;
$R^f$ der Rest einer über die Aminogruppe gebundenen α-Aminocarbonsäure oder eines Esters oder Amids davon;
$R^g$ H oder nieder-Alkyl;
$R^2$ H, $CH_3$, $OCH_3$, $NO_2$, Halogen, $NH_2$, NHCO-phenylen-COOH, $-NHCO(CH_2)_{1-4}-COOH$, $OR^h$, $-CH_2CH_2OR^h$, $-CH_2CH_2OCH_2CH_2OR^h$, oder $-CH_2COOR^h$; und
$R^h$ H oder nieder-Alkyl ist,
sowie Hydrate oder Solvate und physiologisch verwendbare Salze davon.

**2.** Verbindungen nach Anspruch 1 der Formel

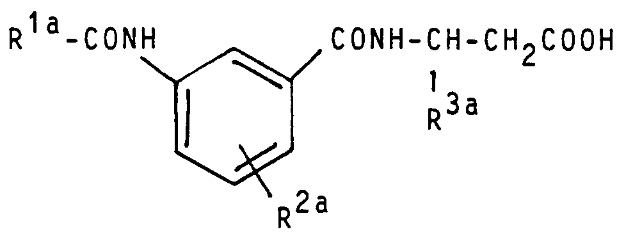

IA

worin $R^{1a}$ eine Gruppe der Formeln R-1, R-2 oder R-3 ist;
$R^{2a}$ H, $-CH_3$, $-OCH_3$ $-NO_2$, Halogen, $-NH_2$, -NHCO-phenylen-COOH oder $-NHCO(CH_2)_{1-4}-COOH$ ist;
$R^{3a}$ H, $-CONH_2$ oder $-COR^i$;
$R^i$ der Rest einer über die Aminogruppe gebundenen α-Aminocarbonsäure ist
und R-1, R-2 und R-3 die oben angegebene Bedeutung haben.

**3.** Verbindungen der Formel I nach Anspruch 1 worin $R^1$ ein Rest der Formel R-1 mit der in Anspruch 1 angegebenen Bedeutung ist.

**4.** Verbindungen nach Anspruch 3, worin $R^1$ (Amino oder Guanidino)-$(CH_2)_{1-7}$- ist oder worin $R^1$-CO der Säurerest von Arginin, Ornithin, $N^2$-Boc-Arginin oder $N^2$-Boc-Ornithin ist.

**5.** Verbindungen der Formel I nach Anspruch 1, worin $R^1$ ein Rest R-2 mit der in Anspruch 1 angegebenen Bedeutung ist.

**6.** Verbindungen nach Anspruch 5 worin $R^1$ α-(Amino oder Guanidino)-(m oder p)-tolyl oder α-Amino-p-tolylmethyl ist.

**7.** Verbindungen der Formel I nach Anspruch 1, worin $R^1$ ein Rest R-3 mit der in Anspruch 1 angegebenen Bedeutung ist.

**8.** Verbindungen nach Anspruch 7 worin $R^1$-CO 3-(p-Amidinophenyl)-DL-alanyl oder p-Amidinobenzoyl ist.

**9.** Verbindungen der Formel I nach Anspruch 1, worin $R^1$ ein Rest R-4 ist.

**10.** Verbindungen der Formel I nach Anspruch 1, worin $R^1$ ein Rest R-5 ist.

**11.** Verbindungen der Formel I nach einem der Ansprüche 1-10, worin $R^3$ Wasserstoff, Carboxyisobutylidenaminocarbonyl, 2-Carboxyphenylaminocarbonyl oder 1-Carboxy-2-(1-naphthyl)äthylidenaminocarbonyl ist.

**12.** Verbindungen der Formel I nach einem der Ansprüche 1-11, worin $R^2$ H, $-CH_3$, $-OCH_3$, $NO_2$ oder -Cl ist.

**13.** Die Verbindungen nach Anspruch 3,
N-[m-(L-Arginylamino)benzoyl]-β-alanin;
N-[m-(L-Arginylamino)benzoyl]-β-alaninformiat;
N-[3-(L-Arginylamino)-p-anisoyl]-β-alaninformiat;
N-[3-(L-Arginylamino)-p-toluoyl]-β-alaninformiat;
N-[m-(6-Aminohexanamido)benzoyl]-β-alanin;
N-[m-(β-Alanylamino)benzoyl]-β-alanin;
N-[m-(8-Aminooctanamido)benzoyl]-β-alanin;
N-[m-(5-Aminovaleramido)benzoyl]-β-alanin;
N-[m-(6-Guanidinohexanamido)benzoyl]-β-alanin;

N-[m-(8-Guanidinooctanamido)benzoyl]-β-alanin;
N-[m-[(N-Amidino-β-alanyl)amino]benzoyl]-β-alanin;
N-[N-[m(-L-Arginylamino)benzoyl]-L-α-aspartyl]-L-valinacetat;
N-[3-(6-Aminohexanamido)-5-nitrobenzoyl] -β-alaninformiat;
N-[5-(6-Aminohexanamido)-2-chlorbenzoyl] -β-alanin;
N-[5-(β-Alanylamino)-2-nitrobenzoyl] -β-alanin;
N-[3-(6-Guanidinohexanamido)-5-nitrobenzoyl] -β-alanin;
N-[5- (6 Guanidinohexanamido)-2-chlorbenzoyl] -β-alanin;
[m- (Boc-Arg-NH)Benzoyl]-Asp-Val;
N-[m-(7-Aminoheptanamido)benzoyl-β-alanin;
N-[m-(5-Guanidinovaleramido)benzoyl]-β-alanin;
N-[m-(7-Guanidinoheptanamido)benzoyl-β-alanin;
N-[m-(4-Guanidinobutyramido)benzoyl]-β-alanin;
N-[m-(4-Aminobutyramido)benzoyl]-β-alanin;
N-[m-[[(S)-$N_2$-(t-Butoxycarbonyl)-L-ornithyl]amino]benzoyl]-β-alaninacetat;
N-[m-(-L-Ornithylamino)benzoyl]-β-alaninformiat;
N-[5-(β-Alanylamino)anthraniloyl]-β-alanin-trifluoracetat;
N-[m-[(N-Amidinoglycyl)amino]benzoyl]-β-alaninformiat;
N-[m-(-L-Lysylamino)benzoyl]-β-alaninformiat;
DL-N-[m-(5-Aminovaleramido)benzoyl]-3-phenyl-β-alaninformiat;
N-[3-(5-Aminovaleramido)-4-chlorbenzoyl]-β-alaninformiat;
N-[3-(5-Aminovaleramido)-p-toluoyl]-β-alanin;
DL-N-[m-(5-Aminovaleramido)benzoyl]-3-(5-indanyl)-β-alanintrifluoracetat;
DL-N-[m-(5-(Guanidinovaleramido)benzoyl]-3-(5-indanyl)-β-alaninformiat;
N-[5-(5-Aminovaleramido)-2-benzyloxy)benzoyl] -β-alanin;
N-[2-(Benzyloxy)-5-(5-Guanidinovaleramido)benzoyl] -β-alanin;
N-[5-[5-Guanidinovaleramido)salicyloyl] -β-alanin.

**14.** Die Verbindungen nach Anspruch 5,
N-[m-(α-Amino-p-toluamido)benzoyl] -β-alanin;
N-[m-(α-Guanidino-p-toluamido)benzoyl] -β-alanin;
N-[m-(α-Amino-m-toluamido)benzoyl] -β-alanin;
N-[m-(α-Guanidino-m-toluamido)benzoyl] -β-alanin;
N-[m-[2-(α-Amino-p-tolyl)acetamido]benzoyl] -β-alanin;
N-[N-[m-[2-(p-Amidinophenyl)acetamido]benzoyl]-L-α-aspartyl]-3-phenyl -L-alanintrifluoracetat;
N-[[m-(α-Amino-p-toluamido)phenyl]acetyl) -β-alanin.

**15.** Die Verbindungen nach Anspruch 7,
N-[m-[[3-(p-Amidinophenyl)-DL-alanyl]amino]benzoyl]-β-alaninacetat;
N-[N-[m-[[-3-(p-Amidinophenyl)-DL-alanyl]amino]benzoyl]-L-α-aspartyl]-3-(-L-naphthyl)-L-alanintrifluoracetat;
N-[N-[m-(p-Amidinobenzamido)benzoyl]-L-α-aspartyl]-3-(1-naphthyl) -L-alanintrifluoracetat;
N-(m-(p-Amidinobenzamido)benzoyl-β-alanin;
N-[m-(p-Amidinobenzamido)benzoyl]-3-(tert.-butoxycarbonyl)-L-alaninmethylester-hydrojodid;
(S)-3-[m-(p-Amidinobenzamido)benzamido]-3-(methoxycarbonyl)propionsäure-trifluoracetat;
N-[m-(p-Amidinobenzamido)benzoyl]-3-(tert.-butoxycarbonyl)-L-alanin;
N-[m-(p-Amidinobenzamido)benzoyl]-L-asparagintrifluoracetat;
(S)-3-[m-(p-Amidinobenzamido)benzamido]-3-(aminocarbonyl)propionsäure;
N-[m-(m-Amidinobenzamido)benzoyl] -β-alanin;
N-[α-(p-Amidinobenzamido) -m-toluoyl]-β-alanin;
N-[α-(p-Amidinobenzamido) -p-toluoyl]-β-alanin;
N-[m-[2-(p-Amidinophenyl)acetamido]benzoyl] -β-alanin;
N-[5-(p-Amidinobenzamido) -2-(benzyloxy)benzoyl-β-alanin;
N-[3-(p-Amidinobenzamido) -4-(benzyloxy)benzoyl]-β-alanin;
N-[3-(p-Amidinobenzamido) -4-hydroxybenzoyl]-β-alanin;
N-[5-(p-Amidinobenzamido)salicyloyl]-β-alanin;
N-[p-(p-Amidinobenzamido)benzoyl]-β-alanin.

**16.** Die Verbindung nach Anspruch 9, N-[m-(trans-4-(Aminomethyl)cyclohexancarboxamido] benzoyl]-β-alanin.

**17.** Die Verbindungen nach Anspruch 10, N-(N-[m-(1-Amidino-4-piperidincarboxamido)benzoyl]-L-α-aspartyl]-3-phenyl-L-alanintrifluoracetat; N-[m-[3-(4-Piperidinyl)propionamido)benzoyl] -β-alanin; N-[m-[3-(1-Amidino-4-piperidinyl)proplonamido] benzoyl]-β-alanintrifluoracetat.

**18.** Verbindungen der Formel

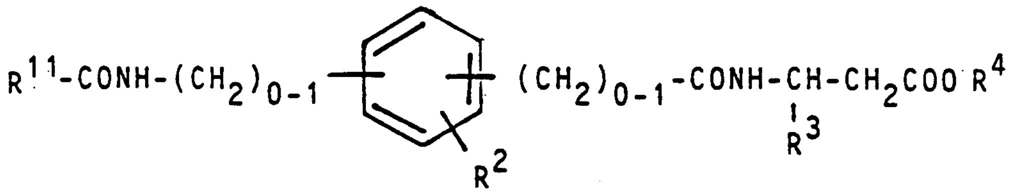

II

worin $R^{11}$ ein Rest der Formeln R-1, R-2, R-3, R-4 oder R-5 gemäss Definition in Anspruch 1 darstelllt, in denen eine gegebenenfalls anwesende Amino-, Amidino- oder Guanidinogruppe in geschützter Form vorliegen kann, $R^4$ Wasserstoff oder eine leicht spaltbare Estergruppe ist und $R^2$ und $R^3$ die in Anspruch 1 angegebene Bedeutung haben; und wobei das Molekül mindestens eine leicht spaltbare Estergruppe oder eine geschützte Amino-, Amidino- oder Guanidinogruppe enthält.

**19.** Verfahren zur Herstellung der Verbindungen der Formel I von Anspruch 1, dadurch gekennzeichnet, dass man aus einer Verbindung der Formel

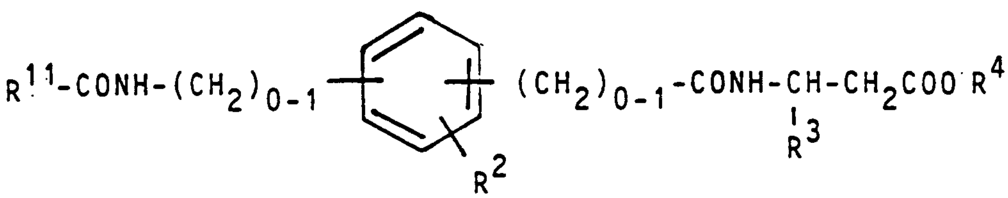

II

worin $R^{11}$ ein Rest der Formeln R-1, R-2, R-3, R-4 oder R-5 gemäss Definition in Anspruch 1 darstelllt, in denen eine gegebenenfalls anwesende Amino-, Amidino- oder Guanidinogruppe in geschützter Form vorliegen kann, $R^4$ Wasserstoff oder eine leicht spaltbare Estergruppe ist und $R^2$ und $R^3$ die in Anspruch 1 angegebene Bedeutung haben; und wobei das Molekül mindestens eine leicht spaltbare Estergruppe oder eine geschützte Amino-, Amidino- oder Guanidinogruppe enthält,
Estergruppe(n) und/oder Amino-, Amidino- oder Guanidinoschutzgruppe(n) abspaltet, gewünschtenfalls eine im Rest $R^1$ enthaltene, reaktionsfähige Gruppe funktionell abwandelt, und gewünschtenfalls eine Verbindung der Formel I in ein physiologisch verträgliches Salz überführt oder ein Salz einer Verbindung der Formel I in die freie Säure oder Base überführt.

**20.** Die Verbindungen der Formel I von Anspruch 1 zur Anwendung als Heilmittel.

**21.** Pharmazeutische Präparate, enthaltend eine Verbindung der Formel I von Anspruch 1 und einen pharmazeutischen Trägerstoff.

**22.** Verwendung einer Verbindung der Formel I von Anspruch 1 zur Herstellung von Arzneimitteln zur Behandlung oder Verhütung von Thrombosen, Hirninfarkt, Herzinfarkt, Arteriosklerose, Entzündungen und Carcinomen.

**Patentansprüche für folgende Vertragsstaaten : ES, GR**

**1.** Verfahren zur Herstellung von Verbindungen der Formel

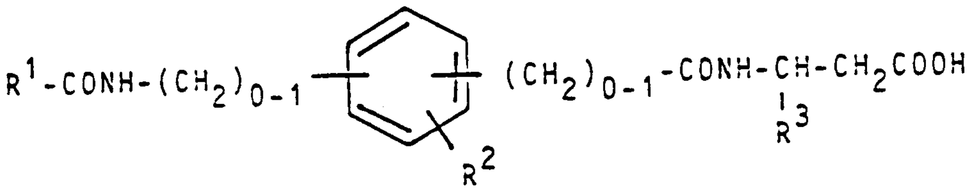

I

worin $R^1$ eine Gruppe der Formel

$-C(H,R^a)-(CH_2)_n-NH-R^b$ (R-1)

$-(T)_{1 \text{ oder } 0}-C_6H_4-CH_2NH-R^c$ (R-2)

$-(T)_{1 \text{ oder } 0}-C_6H_4-(NH)_{1 \text{ oder } 0}-C(NH)NH_2$ (R-3)

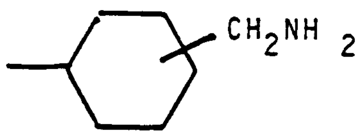

R-4 oder

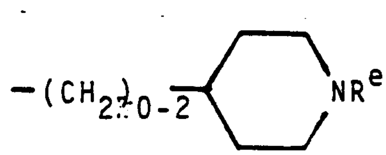

R-5

$R^a$ H, $NH_2$, $-NHCOO-C_{1-4}$-Alkyl, -NH-Z oder $-NHCOCH_2N(Y)-CH_2CH_2NH-Y$, und n 1-6 oder, falls $R^a$ H ist, n auch 0 sein kann,

Y H, Boc oder Z;

$R^b$ H, Amidino oder $-C(NH)-(CH_2)_{0-3}-CH_3$;

$R^c$ H oder Amidino;

T $-CH_2-$, $-CH=CH-$ oder $-C(H,R^d)-CH_2-$ ist;

$R^d$ die gleiche Bedeutung wie $R^a$ hat oder -NHCO-phenyl, -NHCO-phenylen-$N_3$ oder $-NHSO_2$-Aryl ist;

$R^e$ H oder Amidino;

$R^3$ H, $-CONH_2$, $-COR^f$, $COOR^g$ oder Aryl;

$R^f$ der Rest einer über die Aminogruppe gebundenen α-Aminocarbonsäure oder eines Esters oder Amids davon;

$R^g$ H oder nieder-Alkyl;

$R^2$ H, $CH_3$, $OCH_3$, $NO_2$, Halogen, $NH_2$, NHCO-phenylen-COOH, $-NHCO(CH_2)_{1-4}-COOH$, $OR^h$, $-CH_2CH_2OR^h$, $-CH_2CH_2OCH_2CH_2OR^h$, oder $-CH_2COOR^h$; und

$R^h$ H oder nieder-Alkyl ist,

sowie von Hydraten oder Solvaten und physiologisch verwendbaren Salzen davon, dadurch gekennzeichnet, dass man aus einer Verbindung der Formel

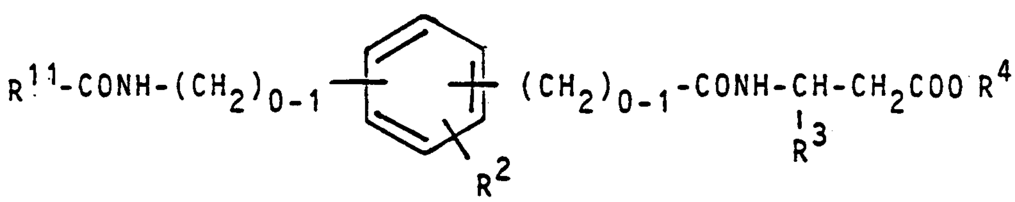

II

worin $R^{11}$ ein Rest der Formeln R-1, R-2, R-3, R-4 oder R-5 gemäss obiger Definition darstellt, in denen eine gegebenenfalls anwesende Amino-, Amidino- oder Guanidinogruppe in geschützter Form vorliegen kann, $R^4$ Wasserstoff oder eine leicht spaltbare Estergruppe ist und $R^2$ und $R^3$ die weiter

oben angegebene Bedeutung haben; und wobei das Molekül mindestens eine leicht spaltbare Estergruppe oder eine geschützte Amino-, Amidino- oder Guanidinogruppe enthält,
Estergruppe(n) und/oder Amino-, Amidino- oder Guanidinoschutzgruppe(n) abspaltet, gewünschtenfalls eine im Rest $R^1$ enthaltene, reaktionsfähige Gruppe funktionell abwandelt, und gewünschtenfalls eine Verbindung der Formel I in ein physiologisch verträgliches Salz überführt oder ein Salz einer Verbindung der Formel I in die freie Säure oder Base überführt.

**2.** Verfahren nach Anspruch 1 der Formel

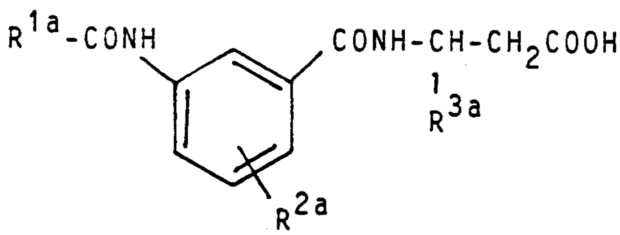

IA

worin $R^{1a}$ eine Gruppe der Formeln R-1, R-2 oder R-3 ist;
$R^{2a}$ H, $-CH_3$, $-OCH_3$ $-NO_2$, Halogen, $-NH_2$, -NHCO-phenylen-COOH oder $-NHCO(CH_2)_{1-4}-COOH$ ist;
$R^{3a}$ H, $-CONH_2$ oder $-COR^i$;
$R^i$ der Rest einer über die Aminogruppe gebundenen α-Aminocarbonsäure ist
und R-1, R-2 und R-3 die oben angegebene Bedeutung haben.

**3.** Verfahren nach Anspruch 1 worin $R^1$ ein Rest der Formel R-1 mit der in Anspruch 1 angegebenen Bedeutung ist.

**4.** Verfahren nach Anspruch 3, worin $R^1$ (Amino oder Guanidino)-$(CH_2)_{1-7}$- ist oder worin $R^1$-CO der Säurerest von Arginin, Ornithin, $N^2$-Boc-Arginin oder $N^2$-Boc-Ornithin ist.

**5.** Verfahren nach Anspruch 1, worin $R^1$ ein Rest R-2 mit der in Anspruch 1 angegebenen Bedeutung ist.

**6.** Verfahren nach Anspruch 5 worin $R^1$ α-(Amino oder Guanidino)-(m oder p)-tolyl oder α-Amino-p-tolylmethyl ist.

**7.** Verfahren nach Anspruch 1, worin $R^1$ ein Rest R-3 mit der in Anspruch 1 angegebenen Bedeutung ist.

**8.** Verfahren nach Anspruch 7 worin $R^1$-CO 3-(p-Amidinophenyl)-DL-alanyl oder p-Amidinobenzoyl ist.

**9.** Verfahren nach Anspruch 1, worin $R^1$ ein Rest R-4 ist.

**10.** Verfahren nach Anspruch 1, worin $R^1$ ein Rest R-5 ist.

**11.** Verfahren nach einem der Ansprüche 1-10, worin $R^3$ Wasserstoff, Carboxyisobutylidenaminocarbonyl, 2-Carboxyphenylaminocarbonyl oder 1-Carboxy-2-(1-naphthyl)äthylidenaminocarbonyl ist.

**12.** Verfahren nach einem der Ansprüche 1-11, worin $R^2$ H, $-CH_3$, $-OCH_3$, $NO_2$ oder -Cl ist.

**13.** Verfahren nach Anspruch 3, in dem man Verbindungen nach Anspruch 3 aus der Gruppe der folgenden herstellt:
N-[m-(L-Arginylamino)benzoyl]-β-alanin;
N-[m-(L-Arginylamino)benzoyl]-β-alaninformiat;
N-[3-(L-Arginylamino)-p-anisoyl]-β-alaninformiat;
N-[3-(L-Arginylamino)-p-toluoyl]-β-alaninformiat;
N-[m-(6-Aminohexanamido)benzoyl]-β-alanin;
N-[m-(β-Alanylamino)benzoyl]-β-alanin;

N-[m-(8-Aminooctanamido)benzoyl]-β-alanin;
N-[m-(5-Aminovaleramido)benzoyl]-β-alanin;
N-[m-(6-Guanidinohexanamido)benzoyl]-β-alanin;
N-[m-(8-Guanidinooctanamido)benzoyl]-β-alanin;
N-[m-[(N-Amidino-β-alanyl)amino]benzoyl]-β-alanin;
N-[N-[m(-L-Arginylamino)benzoyl]-L-α-aspartyl]-L-valinacetat;
N-[3-(6-Aminohexanamido)-5-nitrobenzoyl] -β-alaninformiat;
N-[5-(6-Aminohexanamido)-2-chlorbenzoyl] -β-alanin;
N-[5-(β-Alanylamino)-2-nitrobenzoyl] -β-alanin;
N-[3-(6-Guanidinohexanamido)-5-nitrobenzoyl] -β-alanin;
N-[5-(6-Guanidinohexanamido)-2-chlorbenzoyl] -β-alanin;
[m- (Boc-Arg-NH)Benzoyl]-Asp-Val;
N-[m-(7-Aminoheptanamido)benzoyl-β-alanin;
N-[m-(5-Guanidinovaleramido)benzoyl]-β-alanin;
N-[m-(7-Guanidinoheptanamido)benzoyl-β-alanin;
N-[m-(4-Guanidinobutyramido)benzoyl]-β-alanin;
N-[m-(4-Aminobutyramido)benzoyl]-β-alanin;
N-[m-[[(S)-$N_2$-(t-Butoxycarbonyl)-L-ornithyl]amino]benzoyl]-β-alaninacetat;
N-[m-(-L-Ornithylamino)benzoyl]-β-alaninformiat;
N-[5-(β-Alanylamino)anthraniloyl]-β-alanin-trifluoracetat;
N-[m-[(N-Amidinoglycyl)amino]benzoyl]-β-alaninformiat;
N-[m-(-L-Lysylamino)benzoyl]-β-alaninformiat;
DL-N-[m-(5-Aminovaleramido)benzoyl]-3-phenyl-β-alaninformiat;
N-[3-(5-Aminovaleramido)-4-chlorbenzoyl]-β-alaninformiat;
N-[3-(5-Aminovaleramido)-p-toluoyl]-β-alanin;
DL-N-[m-(5-Aminovaleramido)benzoyl]-3-(5-indanyl)-β-alanintrifluoracetat;
DL-N-[m-(5-(Guanidinovaleramido)benzoyl]-3-(5-indanyl)-β-alaninformiat;
N-[5-(5-Aminovaleramido)-2-benzyloxy)benzoyl] -β-alanin;
N-[2-(Benzyloxy)-5-(5-Guanidinovaleramido)benzoyl] -β-alanin;
N-[5-[5-Guanidinovaleramido)salicyloyl] -β-alanin.

**14.** Verfahren nach Anspruch 5, in dem man Verbindungen nach Anspruch 5 aus der Gruppe der folgenden herstellt:
N-[m-(α-Amino-p-toluamido)benzoyl] -β-alanin;
N-[m-(α-Guanidino-p-toluamido)benzoyl] -β-alanin;
N-[m-(α-Amino-m-toluamido)benzoyl] -β-alanin;
N-[m-(α-Guanidino-m-toluamido)benzoyl] -β-alanin;
N-[m-[2-(α-Amino-p-tolyl)acetamido]benzoyl] -β-alanin;
N-[N-[m-[2-(p-Amidinophenyl)acetamido]benzoyl]-L-α-aspartyl]-3-phenyl -L-alanintrifluoracetat;
N-[[m-(α-Amino-p-toluamido)phenyl]acetyl) -β-alanin.

**15.** Verfahren nach Anspruch 7, in den man Verbindungen nach Anspruch 7 aus der Gruppe der folgenden herstellt:
N-[m-[[3-(p-Amidinophenyl)-DL-alanyl]amino]benzoyl]-β-alaninacetat;
N-[N-[m-[[-3-(p-Amidinophenyl)-DL-alanyl]amino]benzoyl]-L-α-aspartyl]-3-(-L-naphthyl)-L-alanintrifluoracetat;
N-[N-[m-(p-Amidinobenzamido)benzoyl]-L-α-aspartyl]-3-(1-naphthyl) -L-alanintrifluoracetat;
N-(m-(p-Amidinobenzamido)benzoyl-β-alanin;
N-[m-(p-Amidinobenzamido)benzoyl]-3-(tert.-butoxycarbonyl)-L-alaninmethylester-hydrojodid;
(S)-3-[m-(p-Amidinobenzamido)benzamido]-3-(methoxycarbonyl)propionsäure-trifluoracetat;
N-[m-(p-Amidinobenzamido)benzoyl]-3-(tert.-butoxycarbonyl)-L-alanin;
N-[m-(p-Amidinobenzamido)benzoyl]-L-asparagintrifluoracetat;
(S)-3-[m-(p-Amidinobenzamido)benzamido]-3-(aminocarbonyl)propionsäure;
N-[m-(m-Amidinobenzamido)benzoyl] -β-alanin;
N-[α-(p-Amidinobenzamido) -m-toluoyl]-β-alanin;
N-[α-(p-Amidinobenzamido) -p-toluoyl]-β-alanin;
N-[m-[2-(p-Amidinophenyl)acetamido]benzoyl] -β-alanin;
N-[5-(p-Amidinobenzamido) -2-(benzyloxy)benzoyl-β-alanin;

N-[3-(p-Amidinobenzamido) -4-(benzyloxy)benzoyl]-β-alanin;
N-[3-(p-Amidinobenzamido) -4-hydroxybenzoyl]-β-alanin;
N-[5-(p-Amidinobenzamido)salicyloyl]-β-alanin;
N-[p-(p-Amidinobenzamido)benzoyl]-β-alanin.

**16.** Verfahren nach Anspruch 9, in dem man das N-[m-(trans-4-(Aminomethyl)cyclohexancarboxamido] benzoyl]-β-alanin herstellt.

**17.** Verfahren nach Anspruch 10, in dem man Verbindungen nach Anspruch 10 aus der Gruppe der folgenden herstellt:
N-(N-[m-(1-Amidino-4-piperidincarboxamido)benzoyl]-L-α-aspartyl]-3-phenyl-L-alanintrifluoracetat;
N-[m-[3-(4-Piperidinyl)propionamido)benzoyl] -β-alanin;
N-[m-[3-(1-Amidino-4-piperidinyl)proplonamido] benzoyl]-β-alanintrifluoracetat.

**18.** Verfahren nach Anspruch 1, worin man eine Estergruppe mit einer Base verseift, einen Benzylester durch katalytische Hydrierung spaltet, eine geschützte Aminogruppe mit einer Säure bei einer Temperatur bis zu 40 °C spaltet.

**19.** Verfahren zur Herstellung von pharmazeutischen Präparaten, dadurch gekennzeichnet, dass man eine Verbindung der Formel I nach Anspruch 1 oder ein physiologisch verträgliches Salz davon in eine galenische Darreichungsform bringt.

**20.** Verwendung einer Verbindung der Formel I von Anspruch 1 zur Herstellung von Arzneimitteln zur Behandlung oder Verhütung von Thrombosen, Hirninfarkt, Herzinfarkt, Arteriosklerose, Entzündungen und Carcinomen.

**21.** Verbindungen der Formel

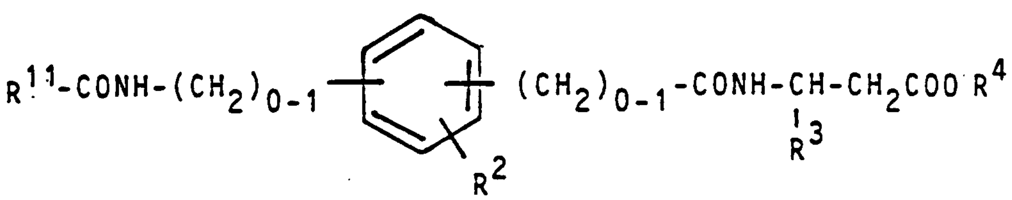

II

worin $R^{11}$ ein Rest der Formeln R-1, R-2, R-3, R-4 oder R-5 gemäss Definition in Anspruch 1 darstelllt, in denen eine gegebenenfalls anwesende Amino-, Amidino- oder Guanidinogruppe in geschützter Form vorliegen kann, $R^4$ Wasserstoff oder eine leicht spaltbare Estergruppe ist und $R^2$ und $R^3$ die in Anspruch 1 angegebene Bedeutung haben; und wobei das Molekül mindestens eine leicht spaltbare Estergruppe oder eine geschützte Amino-, Amidino- oder Guanidinogruppe enthält.

**Claims**
**Claims for the following Contracting States : AT, BE, CH, DE, FR, GB, IT, LI, LU, NL, SE**

**1.** Compounds of the formula

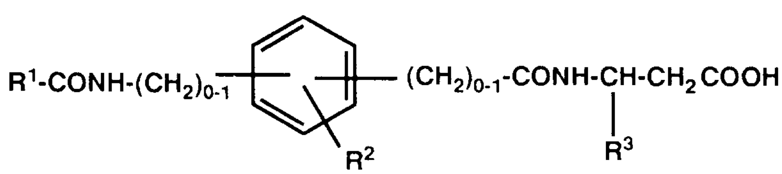

I

wherein $R^1$ is a group of the formula

$-C(H,R^a)-(CH_2)_n-NH-R^b$ (R-1)

$-(T)_{1\ or\ 0}-C_6H_4-CH_2NH-R^c$ (R-2)

$-(T)_{1\ or\ 0}-C_6H_4-(NH)_{1\ or\ 0}-C(NH)NH_2$ (R-3)

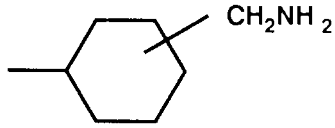

R-4

or

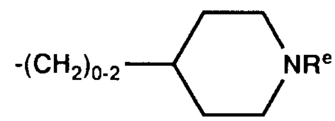

R-5

$R^a$ is H, $NH_2$, $-NHCOO-C_{1-4}$-alkyl, -NH-Z or $-NHCOCH_2N(Y)-CH_2CH_2NH-Y$ and n is 1-6 or, where $R^a$ is H, n can also be 0,

Y is H, Boc or Z;

$R^b$ is H, amidino or $-C(NH)-(CH_2)_{0-3}-CH_3$;

$R^c$ is H or amidino;

T is $-CH_2-$, $-CH=CH-$ or $C(H,R^d)-CH_2-$;

$R^d$ has the same significance as $R^a$ or is -NHCO-phenyl, -NHCO-phenylene-$N_3$ or $-NHSO_2$-aryl;

$R^e$ is H or amidino;

$R^3$ is H, $-CONH_2$, $-COR^f$, $COOR^g$ or aryl;

$R^f$ is the residue of an α-aminocarboxylic acid attached via the amino group or of an ester or amide thereof;

$R^g$ is H or lower-alkyl;

$R^2$ is H, $CH_3$, $OCH_3$, $NO_2$, halogen, $NH_2$, NHCO-phenylene-COOH, $-NHCO(CH_2)_{1-4}-COOH$, $OR^h$, $-CH_2CH_2OR^h$,

$-CH_2CH_2OCH_2CH_2OR^h$ or $-CH_2COOR^h$; and

$R^h$ is H or lower-alkyl,

as well as hydrates or solvates and physiologically usable salts thereof.

2. Compounds according to claim 1 of the formula

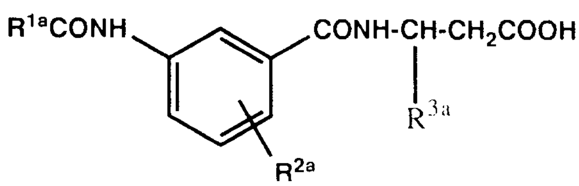

1A

wherein $R^{1a}$ is a group of formula R-1, R-2 or R-3;

$R^{2a}$ is H, $-CH_3$, $-OCH_3$ $-NO_2$, halogen, $-NH_2$, -NHCO-phenylene-COOH or $-NHCO(CH_2)_{1-4}-COOH$;

$R^{3a}$ is H, $-CONH_2$ or $-COR^i$;

$R^i$ is the residue of an α-aminocarboxylic acid attached via the amino group

and R-1, R-2 and R-3 have the significance given above.

3. Compounds of formula I according to claim 1, wherein $R^1$ is a residue of formula R-1 having the significance given in claim 1.

4. Compounds according to claim 3, wherein $R^1$ is (amino or guanidino)-$(CH_2)_{1-7}$- or wherein $R^1$-CO- is the acid residue of arginine, ornithine, $N^2$-Boc-arginine or $N^2$-Boc-ornithine.

5. Compounds of formula I according to claim 1, wherein $R^1$ is a residue R-2 having the significance given in claim 1.

6. Compounds according to claim 5, wherein $R^1$ is α-(amino or guanidino)-(m or p)-tolyl or α-amino-p-tolylmethyl.

7. Compounds of formula I according to claim 1, wherein $R^1$ is a residue R-3 having the significance given in claim 1.

8. Compounds according to claim 7, wherein $R^1$-CO-is 3-(p-amidinophenyl)-DL-alanyl or p-amidinobenzoyl.

9. Compounds of formula I according to claim 1, wherein $R^1$ is a residue R-4.

10. Compounds of formula I according to claim 1, wherein $R^1$ is a residue R-5.

11. Compounds of formula I according to any one of claims 1-10, wherein $R^3$ is hydrogen, carboxyisobutylideneaminocarbonyl, 2-carboxyphenylaminocarbonyl or 1-carboxy-2-(1-naphthyl)-ethylideneaminocarbonyl.

12. Compounds of formula I according to any one of claims 1-11, wherein $R^2$ is H, $-CH_3$, $-OCH_3$, $-NO_2$ or -Cl.

13. The compounds according to claim 3,
N-[m-(L-arginylamino)benzoyl]-β-alanine;
N-[m-(L-arginylamino)benzoyl]-β-alanine formate;
N-[3-(L-arginylamino)-p-anisoyl]-β-alanine formate;
N-[3-(L-arginylamino)-p-toluoyl]-β-alanine formate;
N-[m-(6-aminohexanamido)benzoyl]-β-alanine;
N-[m-(β-alanylamino)benzoyl]-β-alanine;
N-[m-(8-aminooctanamido)benzoyl]-β-alanine;
N-[m-(5-aminovaleramido)benzoyl]-β-alanine;
N-[m-(6-guanidinohexanamido)benzoyl]-β-alanine;
N-[m-(8-guanidinooctanamido)benzoyl]-β-alanine;
N-[m-[(N-amidino-β-alanyl)amino]benzoyl]-β-alanine;
N-[N-[m-(L-arginylamino)benzoyl]-L-α-aspartyl]-L-valine acetate;
N-[3-(6-aminohexanamido)-5-nitrobenzoyl]-β-alanine formate;
N-[5-(6-aminohexanamido)-2-chlorobenzoyl]-β-alanine;
N-[5-(β-alanylamino)-2-nitrobenzoyl]-β-alanine;
N-[3-(6-guanidinohexanamido)-5-nitrobenzoyl]-β-alanine;
N-[5-(6-guanidinohexanamido)-2-chlorobenzoyl]-β-alanine;
[m-(Boc-Arg-NH)benzoyl]-Asp-Val;
N-[m-(7-aminoheptanamido)benzoyl]-β-alanine;
N-[m-(5-guanidinovaleramido)benzoyl]-β-alanine;
N-[m-(7-guanidinoheptanamido)benzoyl]-β-alanine;
N-[m-(4-guanidinobutyramido)benzoyl]-β-alanine;
N-[m-(4-aminobutyramido)benzoyl]-β-alanine;
N-[m-[[(S)-$N_2$-(t-butoxycarbonyl)-L-ornithyl]amino]-benzoyl]-β-alanine acetate;
N-[m-(L-ornithylamino)benzoyl]-β-alanine formate;
N-[5-(β-alanylamino)anthraniloyl]-β-alanine trifluoroacetate;
N-[m-[(N-amidinoglycyl)amino]benzoyl]-β-alanine formate;
N-[m-(L-lysylamino)benzoyl]-β-alanine formate;
DL-N-[m-(5-aminovaleramido)benzoyl]-3-phenyl-β-alanine formate;
N-[3-(5-aminovaleramido)-4-chlorobenzoyl]-β-alanine formate;
N-[3-(5-aminovaleramido)-p-toluoyl]-β-alanine;
DL-N-[m-(5-aminovaleramido)benzoyl]-3-(5-indanyl)-β-alanine trifluoroacetate;
DL-N-[m-(5-guanidinovaleramido)benzoyl]-3-(5 -indanyl)-β-alanine formate;
N-[5-(5-aminovaleramido)-2-(benzyloxy)benzoyl]-β-alanine;
N-[2-(benzyloxy)-5-(5-guanidinovaleramido)benzoyl]-β-alanine;
N-[5-(5-guanidinovaleramido)salicyloyl]-β-alanine.

14. The compounds according to claim 5,
N-[m-(α-amino-p-toluamido)benzoyl]-β-alanine;
N-[m-(α-guanidino-p-toluamido)benzoyl]-β-alanine;

N-[m-(α-amino-m-toluamido)benzoyl]-β-alanine;
N-[m-(α-guanidino-m-toluamido)benzoyl]-β-alanine;
N-[m-[2-(α-amino-p-tolyl)acetamido]benzoyl]-β-alanine;
N-[N-[m-[2-(p-amidinophenyl)acetamido]benzoyl]-L-α-aspartyl]-3-phenyl-L-alanine trifluoroacetate;
N-[[m-(α-amino-p-toluamido)phenyl]acetyl]-β-alanine.

**15.** The compounds according to claim 7,
N-[m-[[3-(p-amidinophenyl)-DL-alanyl]amino]benzoyl]-β-alanine acetate;
N-[N-[m-[[3-(p-amidinophenyl)-DL-alanyl]amino]-benzoyl]-L-α-aspartyl]-3-(1-naphthyl)-L-alanine trifluoroacetate;
N-[N-[m-(p-amidinobenzamido)benzoyl]-L-α-aspartyl]-3-(1-naphthyl)-L-alanine trifluoroacetate;
N-[m-(p-amidinobenzamido)benzoyl]-β-alanine;
N-[m-(p-amidinobenzamido)benzoyl]-3-(t-butoxycarbonyl)-L-alanine methyl ester hydroiodide;
(S)-3-[m-(p-amidinobenzamido)benzamido]-3-(methoxycarbonyl)propionic acid trifluoroacetate;
N-[m-(p-amidinobenzamido)benzoyl]-3-(t-butoxycarbonyl)-L-alanine;
N-[m-(p-amidinobenzamido)benzoyl]-L-asparagine trifluoroacetate;
(S)-3-[m-(p-amidinobenzamido)benzamido]-3-(aminocarbonyl)propionic acid;
N-[m-(m-amidinobenzamido)benzoyl]-β-alanine;
N-[α-(p-amidinobenzamido)-m-toluoyl]-β-alanine;
N-[α-(p-amidinobenzamido)-p-toluoyl]-β-alanine;
N-[m-[2-(p-amidinophenyl)acetamido]benzoyl]-β-alanine;
N-[5-(p-amidinobenzamido)-2-(benzyloxy)benzoyl]-β-alanine;
N-[3-(p-amidinobenzamido)-4-(benzyloxy)benzoyl]-β-alanine;
N-[3-(p-amidinobenzamido)-4-hydroxybenzoyl]-β-alanine;
N-[5-(p-amidinobenzamido)salicyloyl]-β-alanine;
N-[p-(p-amidinobenzamido)benzoyl]-β-alanine.

**16.** The compound according to claim 9,
N-[m-[trans-4-(aminomethyl)cyclohexanecarboxamido]benzoyl]-β-alanine.

**17.** The compounds according to claim 10,
N-[N-[m-(1-amidino-4-piperidinecarboxamido)benzoyl]-L-α-aspartyl]-3-phenyl-L-alanine trifluoroacetate;
N-[m-[3-(4-piperidinyl)propionamido]benzoyl]-β-alanine;
N-[m-[3-(1-amidino-4-piperidinyl)propionamido]benzoyl]-β-alanine trifluoroacetate.

**18.** Compounds of the formula

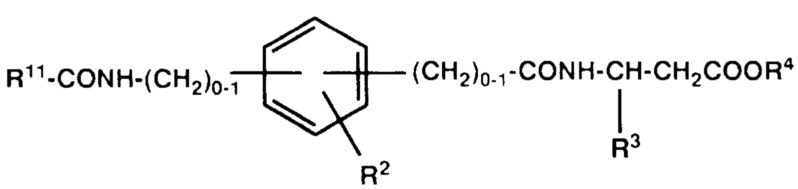

II

wherein $R^{11}$ represents a residue of formula R-1, R-2, R-3, R-4 or R-5 in accordance with the definition in claim 1 in which an optionally present amino, amidino or guanidino group can be present in protected form, $R^4$ is hydrogen or a readily cleavable ester group and $R^2$ and $R^3$ have the significance given in claim 1 and wherein the molecule contains at least one readily cleavable ester group or a protected amino, amidino or guanidino group.

**19.** A process for the manufacture of the compounds of formula I of claim 1, characterized by cleaving off ester group(s) and/or amino, amidino or guanidino protecting group(s) from a compound of the formula

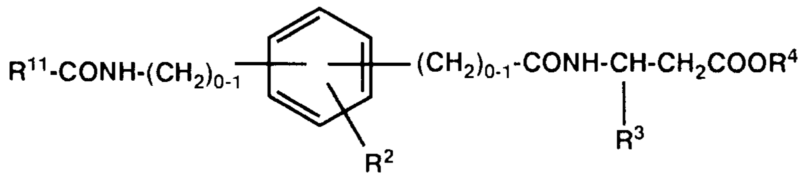

II

wherein $R^{11}$ represents a residue of formula R-1, R-2, R-3, R-4 or R-5 in accordance with the definition in claim 1 in which an optionally present amino, amidino or guanidino group can be present in protected form, $R^4$ is hydrogen or a readily cleavable ester group and $R^2$ and $R^3$ have the significance given earlier; and whereby the molecule contains at least one readily cleavable ester group or a protected amino, amidino or guanidino group,
if desired, functionally modifying a reactive group present in the residue $R^1$ and, if desired, converting a compound of formula I into a physiologically compatible salt or converting a salt of a compound of formula I into the free acid or base.

**20.** The compounds of formula I of claim 1 for use as medicaments.

**21.** Pharmaceutical preparations containing a compound of formula I of claim 1 and a pharmaceutical carrier material.

**22.** The use of a compound of formula I of claim 1 for the manufacture of medicaments for the treatment or prevention of thromboses, stroke, cardiac infarct, arteriosclerosis, inflammations and carcinomas.

**Claims for the following Contracting States : ES, GR**

**1.** A process for the manufacture of compounds of the formula

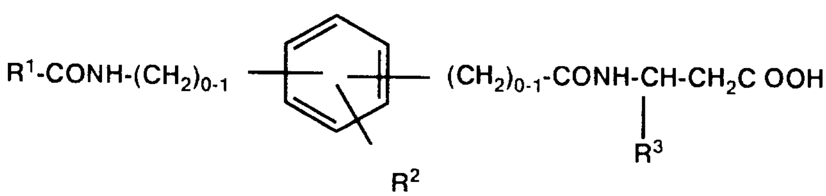

I

wherein $R^1$ is a group of the formula

$-C(H,R^a)-(CH_2)_n-NH-R^b$ (R-1)

$-(T)_{1\ or\ 0}-C_6H_4-CH_2NH-R^c$ (R-2)

$-(T)_{1\ or\ 0}-C_6H_4-(NH)_{1\ or\ 0}-C(NH)NH_2$ (R-3)

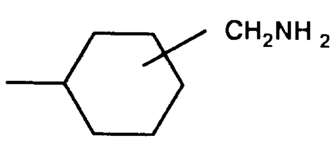

R-4

or

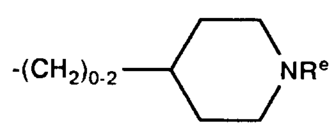

R-5

$R^a$ is H, $NH_2$, $-NHCOO-C_{1-4}$-alkyl, -NH-Z or $-NHCOCH_2N(Y)-CH_2CH_2NH-Y$ and n is 1-6 or, where $R^a$ is H, n can also be 0,
Y is H, Boc or Z;

$R^b$ is H, amidino or $-C(NH)-(CH_2)_{0-3}-CH_3$;
$R^c$ is H or amidino;
T is $-CH_2-$, $-CH=CH-$ or $-C(H,R^d)-CH_2-$;
$R^d$ has the same significance as $R^a$ or is -NHCO-phenyl, -NHCO-phenylene-$N_3$ or -$NHSO_2$-aryl;
$R^e$ is H or amidino;
$R^3$ is H, $-CONH_2$, $-COR^f$, $COOR^g$ or aryl;
$R^f$ is the residue of an α-aminocarboxylic acid attached via the amino group or of an ester or amide thereof;
$R^g$ is H or lower-alkyl;
$R^2$ is H, $CH_3$, $OCH_3$, $NO_2$, halogen, $NH_2$, NHCO-phenylene-COOH, $-NHCO(CH_2)_{1-4}-COOH$, $OR^h$, $-CH_2CH_2OR^h$, $-CH_2CH_2OCH_2CH_2OR^h$ or $-CH_2COOR^h$; and
$R^h$ is H or lower-alkyl,
as well as hydrates or solvates and physiologically usable salts thereof, characterized by cleaving off ester group(s) and/or amino, amidino or guanidino protecting group(s) from a compound of the formula

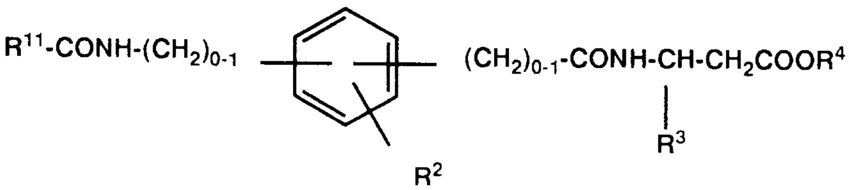

II

wherein $R^{11}$ represents a residue of formula R-1, R-2, R-3, R-4 or R-5 in accordance with the above definition in which an optionally present amino, amidino or guanidino group can be present in protected form, $R^4$ is hydrogen or a readily cleavable ester group and $R^2$ and $R^3$ have the significance given above; and whereby the molecule contains at least one readily cleavable ester group or a protected amino, amidino or guanidino group,
if desired, functionally modifying a reactive group present in the residue $R^1$ and, if desired, converting a compound of formula I into a physiologically compatible salt or converting a salt of a compound of formula I into the free acid or base.

2. A process according to claim 1 of the formula

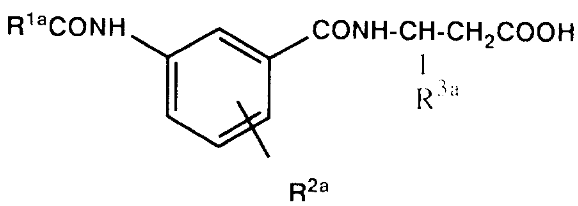

IA

wherein $R^{1a}$ is a group of formula R-1, R-2 or R-3;
$R^{2a}$ is H, $-CH_3$, $-OCH_3$ $-NO_2$, halogen, $-NH_2$, -NHCO-phenylene-COOH or $-NHCO(CH_2)_{1-4}-COOH$;
$R^{3a}$ is H, $-CONH_2$ or $-COR^i$;
$R^i$ is the residue of an α-aminocarboxylic acid attached via the amino group
and R-1, R-2 and R-3 have the significance given above.

3. A process according to claim 1, wherein $R^1$ is a residue of formula R-1 having the significance given in claim 1.

4. A process according to claim 3, wherein $R^1$ is (amino or guanidino)-$(CH_2)_{1-7}$- or wherein R-CO- is the acid residue of arginine, ornithine, $N^2$-Boc-arginine or $N^2$-Boc-ornithine.

5. A process according to claim 1, wherein $R^1$ is a residue R-2 having the significance given in claim 1.

**6.** A process according to claim 5, wherein $R^1$ is $\alpha$-(amino or guanidino)-(m or p)-tolyl or $\alpha$-amino-p-tolylmethyl.

**7.** A process according to claim 1, wherein $R^1$ is a residue R-3 having the significance given in claim 1.

**8.** A process according to claim 7, wherein $R^1$-CO-is 3-(p-amidinophenyl)-DL-alanyl or p-amidinobenzoyl.

**9.** A process according to claim 1, wherein $R^1$ is a residue R-4.

**10.** A process according to claim 1, wherein $R^1$ is a residue R-5.

**11.** A process according to any one of claims 1-10, wherein $R^3$ is hydrogen, carboxyisobutylideneaminocarbonyl, 2-carboxyphenylaminocarbonyl or 1-carboxy-2-(1-naphthyl)ethylidene-aminocarbonyl.

**12.** A process according to any one of claims 1-11, wherein $R^2$ is H, $-CH_3$, $-OCH_3$, $-NO_2$ or -Cl.

**13.** A process according to claim 3, in which compounds according to claim 3 from the following group are manufactured:
N-[m-(L-arginylamino)benzoyl]-$\beta$-alanine;
N-[m-(L-arginylamino)benzoyl]-$\beta$-alanine formate;
N-[3-(L-arginylamino)-p-anisoyl]-$\beta$-alanine formate;
N-[3-(L-arginylamino)-p-toluoyl]-$\beta$-alanine formate;
N-[m-(6-aminohexanamido)benzoyl]-$\beta$-alanine;
N-[m-($\beta$-alanylamino)benzoyl]-$\beta$-alanine;
N-[m-(8-aminooctanamido)benzoyl]-$\beta$-alanine;
N-[m-(5-aminovaleramido)benzoyl]-$\beta$-alanine;
N-[m-(6-guanidinohexanamido)benzoyl]-$\beta$-alanine;
N-[m-(8-guanidinooctanamido)benzoyl]-$\beta$-alanine;
N-[m-[(N-amidino-$\beta$-alanyl)amino]benzoyl]-$\beta$-alanine;
N-[N-[m-(L-arginylamino)benzoyl]-L-$\alpha$-aspartyl]-L-valine acetate;
N-[3-(6-aminohexanamido)-5-nitrobenzoyl]-$\beta$-alanine formate;
N-[5-(6-aminohexanamido)-2-chlorobenzoy]-$\beta$-alanine;
N-[5-($\beta$-alanylamino)-2-nitrobenzoyl]-$\beta$-alanine;
N-[3-(6-guanidinohexanamido)-5-nitrobenzoy]-$\beta$-alanine;
N-[5-(6-guanidinohexanamido)-2-chlorobenzoyl]-$\beta$-alanine;
[m-(Boc-Arg-NH)benzoyl]-Asp-Val;
N-[m-(7-aminoheptanamido)benzoyl]-$\beta$-alanine;
N-[m-(5-guanidinovaleramido)benzoyl]-$\beta$-alanine;
N-[m-(7-guanidinoheptanamido)benzoyl]-$\beta$-alanine;
N-[m-(4-guanidinobutyramido)benzoyl]-$\beta$-alanine;
N-[m-(4-aminobutyramido)benzoyl]-$\beta$-alanine;
N-[m-[[(S)-$N_2$-(t-butoxycarbonyl)-L-ornithyl]amino]-benzoyl]-$\beta$-alanine acetate;
N-[m-(L-ornithylamino)benzoyl]-$\beta$-alanine formate;
N-[5-($\beta$-alanylamino)anthraniloyl]-$\beta$-alanine trifluoroacetate;
N-[m-[(N-amidinoglycyl)amino]benzoyl]-$\beta$-alanine formate;
N-[m-(L-lysylamino)benzoyl]-$\beta$-alanine formate;
DL-N-[m-(5-aminovaleramido)benzoyl]-3-phenyl-$\beta$-alanine formate;
N-[3-(5-aminovaleramido)-4-chlorobenzoyl]-$\beta$-alanine formate;
N-[3-(5-aminovaleramido)-p-toluoyl]-$\beta$-alanine;
DL-N-[m-(5-aminovaleramido)benzoyl]-3-(5-indanyl)-$\beta$-alanine trifluoroacetate;
DL-N-[m-(5-guanidinovaleramido)benzoyl]-3-(5-indanyl)-$\beta$-alanine formate;
N-[5-(5-aminovaleramido)-2-(benzyloxy)benzoyl]-$\beta$-alanine;
N-[2-(benzyloxy)-5-(5-guanidinovaleramido)benzoyl]-$\beta$-alanine;
N-[5-(5-guanidinovaleramido)salicyloyl]-$\beta$-alanine.

**14.** A process according to claim 5, in which compounds according to claim 5 from the following group are manufactured:
N-[m-($\alpha$-amino-p-toluamido)benzoyl]-$\beta$-alanine;

N-[m-(α-guanidino-p-toluamido)benzoyl]-β-alanine;
N-[m-(α-amino-m-toluamido)benzoyl]-β-alanine;
N-[m-(α-guanidino-m-toluamido)benzoyl]-β-alanine;
N-[m-[2-(α-amino-p-tolyl)acetamido]benzoyl]-β-alanine;
N-[N-[m-[2-(p-amidinophenyl)acetamido]benzoyl]-L-α-aspartyl]-3-phenyl-L-alanine trifluoroacetate;
N-[[m-(α-amino-p-toluamido)phenyl]acetyl]-β-alanine.

**15.** A process according to claim 7, in which compounds according to claim 7 from the following group are manufactured:
N-[m-[[3-(p-amidinophenyl)-DL-alanyl]amino]benzoyl)-β-alanine acetate;
N-[N-[m-[[3-(p-amidinophenyl)-DL-alanyl]amino]-benzoyl]-L-a-aspartyl]-3-(1-naphthyl)-L-alanine trifluoroacetate;
N-[N-[m-(p-amidinobenzamido)benzoyl]-L-a-asparty]-3-(1-naphthyl)-L-alanine trifluoroacetate;
N-[m-(p-amidinobenzamido)benzoyl]-β-alanine;
N-[m-(p-amidinobenzamido)benzoyl]-3-(t-butoxycarbonyl)-L-alanine methyl ester hydroiodide;
(S)-3-[m-(p-amidinobenzamido)benzamido]-3-(methoxy-carbonyl)propionic acid trifluoroacetate;
N-[m-(p-amidinobenzamido)benzoyl]-3-(t-butoxycarbony)-L-alanine;
N-[m-(p-amidinobenzamido)benzoyl]-L-asparagine trifluoroacetate;
(S)-3-[m-(p-amidinobenzamido)benzamido]-3-(aminocarbonyl)propionic acid;
N-[m-(m-amidinobenzamido)benzoyl]-β-alanine;
N-[α-(p-amidinobenzamido)-m-toluoyl]-β-alanine;
N-[α-(p-amidinobenzamido)-p-toluoyl]-β-alanine;
N-[m-[2-(p-amidinophenyl)acetamido]benzoyl]-β-alanine;
N-[5-(p-amidinobenzamido)-2-(benzyloxy)benzoyl]-β-alanine;
N-[3-(p-amidinobenzamido)-4-(benzyloxy)benzoyl]-β-alanine;
N-[3-(p-amidinobenzamido)-4-hydroxybenzoyl]-β-alanne;
N-[5-(p-amidinobenzamido)salicyloyl]-β-alanine;
N-[p-(p-amidinobenzamido)benzoyl]-β-alanine.

**16.** A process according to claim 9, in which N-[m-[trans-4-(aminomethyl)cyclohexanecarboxamido]-benzoyl]-β-alanine is manufactured.

**17.** A process according to claim 10, in which compounds according to claim 10 from the following group are manufactured:
N-[N-[m-(1-amidino-4-piperidinecarboxamido)benzoyl]-L-α-aspartyl]-3-phenyl-L-alanine trifluoroacetate;
N-[m-[3-(4-piperidinyl)propionamido)benzoyl]-β-alanine;
N-[m-[3-(1-amidino-4-piperidinyl)propionamido]benzoy]-β-alanine trifluoroacetate.

**18.** A process according to claim 1, wherein an ester group is saponified with a base, a benzyl ester is cleaved by catalytic hydrogenation, a protected amino group is cleaved with an acid at a temperature up to 40 °C.

**19.** A process for the manufacture of pharmaceutical preparations, characterized by bringing a compound of formula I according to claim 1 or a physiologically compatible salt thereof into a galenical administration form.

**20.** The use of a compound of formula I of claim 1 for the manufacture of medicaments for the treatment or prevention of thromboses, cardiac infarct, arteriosclerosis, inflammations and carcinomas.

**21.** Compounds of the formula

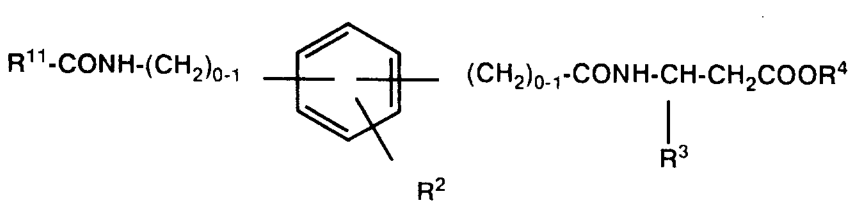

II

wherein $R^{11}$ represents a residue of formula R-1, R-2, R-3, R-4 or R-5 in accordance with the definition in claim 1 in which an optionally present amino, amidino or guanidino group can be present in protected form, $R^4$ is hydrogen or a readily cleavable ester group and $R^2$ and $R^3$ have the significance given in claim 1; and whereby the molecule contains at least one readily cleavable ester group or a protected amino, amidino or guanidino group.

**Revendications**
**Revendications pour les Etats contractants suivants : AT, BE, CH, DE, FR, GB, IT, LI, LU, NL, SE**

**1.** Composés de formule

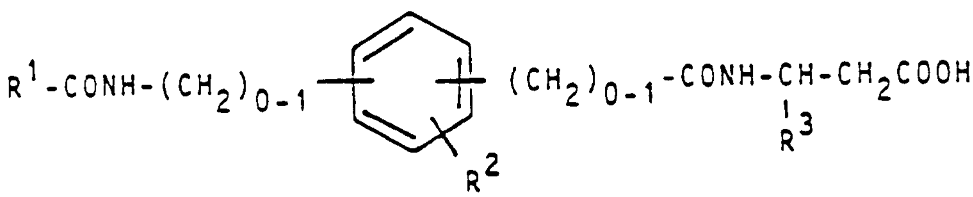

I

où $R^1$ est un groupe de formule

$-C(H,R^a)-(CH_2)_n-NH-R^b$ (R-1)

$-(T)_{1\ ou\ O}-C_6H_4-CH_2NH-R^c$ (R-2)

$-(T)_{1\ ou\ O}-C_6H_4-(NH)_{1\ ou\ O}-C(NH)NH_2$ (R-2)

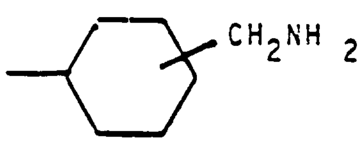 R-4 ou 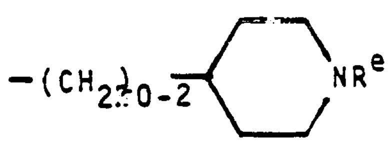 R-5

$R^a$ est H, $NH_2$, -NHCOO-$C_{1-4}$-Alkyle, -NH-Z ou $-NHCOCH_2N(Y)-CH_2CH_2NH-Y$ et n est compris entre 1 et 6 ou, lorsque $R^a$ est H, n peut être aussi égal à 0,

Y est H, Boc ou Z;

$R^b$ est H, un amidino ou $-C(NH)-(CH_2)_{O-3}-CH_3$;

$R^c$ est H ou un amidino;

T est $-CH_2-$, -CH = CH- ou $-C(H,R^d)-CH_2-$;

$R^d$ a la même signification que $R^a$ ou est -NHCO-phényle, -NHCO-phénylène-$N_3$ ou -$NHSO_2$-aryle;

$R^e$ est H ou un amidino;

$R^3$ est H, $-CONH_2$, $-COR^f$, $COOR^g$ ou un aryle;

$R^f$ est le reste d'un acide $\alpha$-aminé lié par le groupe amino ou de l'un de ses esters ou de ses amides;

$R^g$ est H ou un alkyle inférieur;

$R^2$ est H, $CH_3$, $OCH_3$, $NO_2$, un halogène, $NH_2$, NHCO-phénylène-COOH, $-NHCO(CH_2)_{1-4}$-COOH, $OR^h$, $-CH_2CH_2OR^h$, $-CH_2CH_2OCH_2CH_2OR^h$ ou $-CH_2COOR^h$; et

$R^h$ est H ou un alkyle inférieur,

ainsi que leurs hydrates ou solvates et leurs sels physiologiquement utilisables.

2. Composés selon la revendication 1 de formule

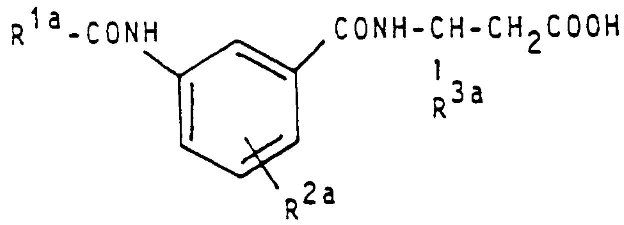

IA

où $R^{1a}$ est un groupe de formules R-1, R-2 ou R-3;
$R^{2a}$ est H, $-CH_3$, $-OCH_3$, $-NO_2$, un halogène, $-NH_2$, -NHCO-phénylène-COOH ou $-NHCO(CH_2)_{1-4}$-COOH;
$R^{3a}$ est H, $-CONH_2$ ou $-COR^i$;
$R^i$ est le reste d'un acide $\alpha$-aminé lié par le groupe amino
et R-1, R-2 et R-3 ont les significations données ci-dessus.

3. Composés de formule I selon la revendication 1 où $R^1$ est un reste de formule R-1 ayant la signification donnée dans la revendication 1.

4. Composés selon la revendication 3 où $R^1$ est un (amino ou guanidino)-$(CH_2)_{1-7}$- ou où $R^1$-CO est le reste acide de l'arginine, de l'ornithine, de la $N^2$-Boc-arginine ou de la $N^2$-Boc-ornithine.

5. Composés de formule I selon la revendication 1, où $R^1$ est un reste R-2 ayant la signification donnée dans la revendication 1.

6. Composés selon la revendication 5, où $R^1$ est un $\alpha$-(amino ou guanidino)-(m ou p)-tolyle ou l'$\alpha$-amino-p-tolylméthyle.

7. Composés de formule I selon la revendication 1, où $R^1$ est un reste R-3 ayant la signification donnée dans la revendication 1.

8. Composés selon la revendication 7 où $R^1$-CO est le 3-(p-amidinophényl)-DL-alanyle ou le p-amidinobenzoyle.

9. Composés de formule I selon la revendication 1, où $R^1$ est un reste R-4.

10. Composés de formule I selon la revendication 1, où $R^1$ est un reste R-5.

11. Composés de formule I selon l'une des revendications 1 à 10, où $R^3$ est l'hydrogène, un carboxyisobutylidèneaminocarbonyle, le 2-carboxyphénylaminocarbonyle ou le 1-carboxy-2-(1-naphtyl)-éthylidèneaminocarbonyle.

12. Composés de formule I selon l'une des revendications 1 à 11, où $R^2$ est H, $-CH_3$, $-OCH_3$, $NO_2$ ou -Cl.

13. Les composés selon la revendication 3 :
la N-[m-(L-arginylamino)benzoyl]-$\beta$-alanine;
le formiate de N-[m-(L-arginylamino)benzoyl]-$\beta$-alanine;
le formiate de N-[3-(L-arginylamino)-p-anisoyl]-$\beta$-alanine;
le formiate de N-[3-(L-arginylamino)-p-toluoyl]-$\beta$-alanine;
la N-[m-(6-aminohexanamido)benzoyl]-$\beta$-alanine;
la N-[m-($\beta$-alanylamino)benzoyl]-$\beta$-alanine;
la N-[m-(8 -aminooctanamido)benzoyl]-$\beta$-alanine;
la N-[m-(5-aminovaléramido)benzoyl]-$\beta$-alanine;
la N-[m-(6-guanidinohexanamido)benzoyl]-$\beta$-alanine;
la N-[m-(8-guanidinooctanamido)benzoyl]-$\beta$-alanine;

la N-[m-[(N-amidino-β-alanyl)amino]benzoyl]-β-alanine;
l'acétate de N-[N-[m-(L-arginylamino)benzoyl]-L-α-aspartyl]-L-valine;
le formiate de N-[3-(6-aminohexanamido)-5-nitrobenzoyl]-β-alanine;
la N-[5-(6-aminohexanamido)-2-chlorobenzoyl]-β-alanine;
la N-[5-(β-alanylamino)-2-nitrobenzoyl]-β-alanine;
la N-[3-(6-guanidinohexanamido)-5-nitrobenzoyl]-β-alanine;
la N-[5-(6-guanidinonexanamido)-2-chlorobenzoyl]-β-alanine;
le [m-(Boc-Arg-NH)benzoyl]-Asp-Val;
la N-[m-(7-aminoheptanamido)benzoyl]-β-alanine;
la N-[m-(5-guanidinovaléramido)benzoyl]-β-alanine;
la N-[m-(7-guanidinoheptanamido)benzoyl]-β-alanine;
la N-[m-(4-guanidinobutyramido)benzoyl]-β-alanine;
la N-[m-(4-aminobutyramido)benzoyl]-β-alanine;
l'acétate de N-[m-[[(S)-$N_2$-(t-butoxycarbonyl)-L-ornithyl] amino]benzoyl]-β-alanine;
le formiate de N-[m-(L-ornithylamino)benzoyl]-β-alanine;
le trifluoroacétate de N-[5-(β-alanylamino)anthraniloyl] -β-alanine;
le formiate de N-[m-[(N-amidinoglycyl)amino]benzoyl]-β-alanine;
le formiate de N-[m-(L-lysylamino)benzoyl]-β-alanine;
le formiate de DL-N-[m-(5-aminovaléramido)benzoyl]-3-phényl-β-alanine;
le formiate de N-[3-(5-aminovaléramido)-4-chlorobenzoyl]-β-alanine;
la N-[3-(5-aminovaléramido)-p-toluoyl]-β-alanine;
le trifluoroacétate de DL-N-[m-(5-aminovaléramido)benzoyl] -3-(5-indanyl)-β-alanine;
le formiate de DL-N-[m-(5-guanidinovaléramido)benzoyl]-3-(5-indanyl)-β-alanine;
la N-[5-(5-aminovaléramido)-2-benzyloxy)benzoyl]-β-alanine;
la N-[2-(benzyloxy)-5-(5-guanidinovaléramido)benzoyl]-β-alanine;
la N-[5-(5-guanidinovaléramido)salicyloyl]-β-alanine.

**14.** Les composés selon la revendication 5 :
la N-[m-(α-amino-p-toluamido)benzoyl]-β-alanine;
la N-[m-(α-guanidino-p-toluamido)benzoyl]-β-alanine;
la N-[m-(α-amino-m-toluamido)benzoyl]-β-alanine;
la N-[m-(α-guanidino-m-toluamido)benzoyl]-β-alanine;
la N-[m-[2-(α-amino-p-tolyl)acétamido]benzoyl]-β-alanine;
le trifluoroacétate de N-[N-[m-[2-(p-amidinophényl)acétamido]benzoyl]-L-α-aspartyl]-3-phényl-L-alanine;
la N-[[m-(α-amino-p-toluamido)phényl]acétyl]-β-alanine.

**15.** Les composés selon la revendication 7 :
l'acétate de N-[m-[[3-(p-amidinophényl)-DL-alanyl]amino] benzoyl]-β-alanine;
le trifluoroacétate de N-[N-[m-[[3-(p-amidinophényl)-DL-alanyl]amino]benzoyl]-L-α-aspartyl]-3-(1-naphtyl)-L-alanine;
le trifluoroacétate de N-[N-[m-(p-amidinobenzamido)benzoyl]-L-α-aspartyl]-3-(1-naphtyl)-L-alanine;
la N-[m-(p-amidinobenzamido)benzoyl]-β-alanine;
l'iodhydrate de l'ester méthyliaue de la N-[m-(p-amidinobenzamido)benzoyl]-3-(tert-butoxycarbonyl)-L-alanine;
le trifluoroacétate de l'acide (S)-3-[m-(p-amidinobenzamido)benzamido]-3-(méthoxycarbonyl)-propionique;
la N-[m-(p-amidinobenzamido)benzoyl]-3-(tert-butoxycarbonyl)-L-alanine;
le trifluoracétate de N-[m-(p-amidinobenzamido)benzoyl]-L-asparagine;
l'acide (S)-3-[m-(p-amidinobenzamido)benzamido]-3-(aminocarbonyl)propionique;
la N-[m-(m-amidinobenzamido)benzoyl]-β-alanine;
la N-[α-(p-amidinobenzamido)-m-toluoyl]-β-alanine;
la N-[α-(p-amidinobenzamido)-p-toluoyl]-β-alanine;
la N-[m-[2-(p-amidinophényl)acétamido]benzoyl]-β-alanine;
la N-[5-(p-amidinobenzamido)-2-(benzyloxy)benzoyl]-β-alanine;
la N-[3-(p-amidinobenzamido)-4-(benzyloxy)benzoyl]-β-alanine;
la N-[3-(p-amidinobenzamido)-4-hydroxybenzoyl]-β-alanine;
la N-[5-(p-amidinobenzamido)salicyloyl]-β-alanine;
la N-[p-(p-amidinobenzamido)benzoyl]-β-alanine.

**16.** Le composé selon la revendication 9,
la N-[m-[trans-4-(aminométhyl)cyclonexanecarboxamido] benzoyl]-$\beta$-alanine.

**17.** Les composés selon la revendication 10 :
le trifluoroacétate de N-[N-[m-(1-amidino-4-pipéridinecarboxamido)benzoyl]-L-$\alpha$-aspartyl]-3-phényl-L-alanine;
la N-[m-[3-(4-pipéridinyl)propionamido)benzoyl]-$\beta$-alanine;
le trifluoroacétate de N-[m-[3-(1-amidino-4-pipéridinyl) propionamido]benzoyl]-$\beta$-alanine.

**18.** Composés de formule

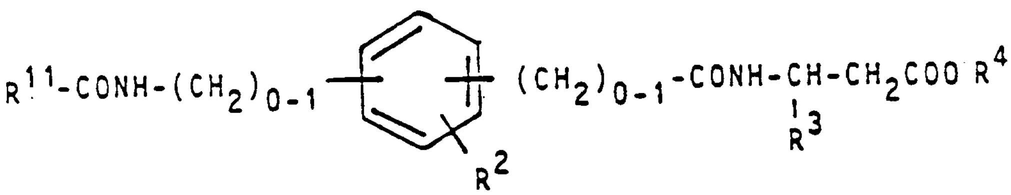

II

où $R^{11}$ représente un reste de formules R-1, R-2, R-3, R-4 ou R-5 conformes aux définitions données dans la revendication 1, dans laquelle un groupe amino, amidino ou guanidino éventuellement présent peut se présenter sous forme protégée, $R^4$ est l'hydrogène, ou un groupe ester facilement clivable et $R^2$ et $R^3$ ont les significations données dans la revendication 1; et où la molécule contient au moins un groupe ester facilement clivable ou un groupe amino, amidino ou guanidino protégé.

**19.** Procédé pour la préparation des composés de formule I de la revendication 1, caractérisé en ce que l'on clive sur un composé de formule

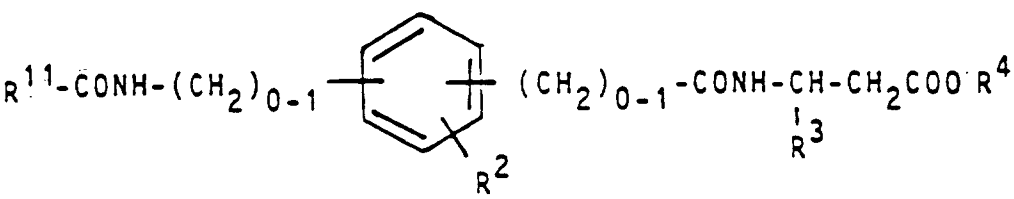

II

où $R^{11}$ représente un reste de formules R-1, R-2, R-3, R-4 ou R-5 conformes aux définitions données dans la revendication 1, dans laquelle un groupe amino, amidino ou guanidino éventuellement présent peut se présenter sous forme protégée, $R^4$ est l'hydrogène ou un groupe ester facilement clivable et $R^2$ et $R^3$ ont la signification donnée dans la revendication 1 et où la molécule contient au moins un groupe ester facilement clivable ou un groupe amino, amidino ou guanidino protégé
le ou les groupe(s) ester(s) et/ou le ou les groupe(s) protecteur(s) de l'amino, de l'amidino ou du guanidino, en ce que, si désiré, l'on modifie fonctionnellement un groupe réactif contenu dans le reste $R^1$ et, en ce que, si désiré, l'on transforme un composé de formule I en un sel physiologiquement compatible ou un sel d'un composé de formule I en un acide ou en une base libre.

**20.** Les composés de formule I de la revendication 1 destinés à être utilisés comme médicaments.

**21.** Préparations pharmaceutiques contenant un composé de formule I de la revendication 1 et un support pharmaceutique.

**22.** Utilisation d'un composé de formule I de la revendication 1 pour la préparation de médicaments destinés au traitement ou à la prévention des thromboses, des attaques cérébrales, des infarctus du myocarde, de l'artériosclérose, des inflammations et des carcinomes.

**Revendications pour les Etats contractants suivants : ES, GR**

**1.** Procédé pour la préparation des composés de formule

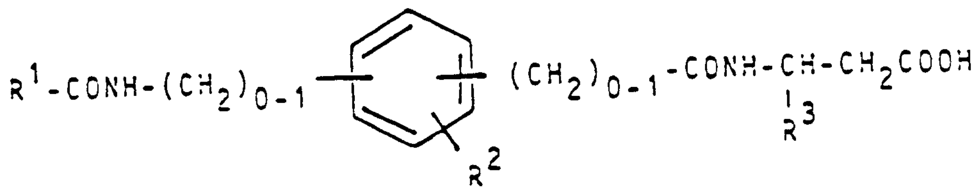 I

où $R^1$ est un groupe de formule

$-C(H,R^a)-(CH_2)_n-NH-R^b$ (R-1)

$-(T)_{1\ ou\ O}-C_6H_4-CH_2NH-R^c$ (R-2)

$-(T)_{1\ ou\ O}-C_6H_4-(NH)_{1\ ou\ O}-C(NH)NH_2$ (R-3)

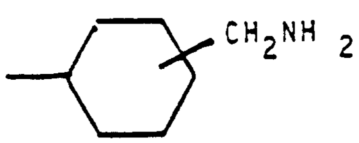 R-4 ou 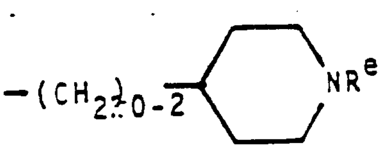 R-5

$R^a$ est H, $NH_2$, $-NHCOO-C_{1-4}$-Alkyle, -NH-Z ou $-NHCOCH_2N(Y)-CH_2CH_2NH-Y$ et n est compris entre 1 et 6 ou, lorsque $R^a$ est H, n peut être aussi égal à 0,

Y est H, Boc ou Z;

$R^b$ est H, un amidino ou $-C(NH)-(CH_2)_{O-3}-CH_3$;

$R^6$ est H ou un amidino;

T est $-CH_2-$, $-CH=CH-$ ou $-C(H,R^d)-CH_2-$;

$R^d$ a la même signification que $R^a$ ou est -NHCO-phényle, -NHCO-phénylène-$N_3$ ou $-NHSO_2$-aryle;

$R^e$ est H ou un amidino;

$R^3$ est H, $-CONH_2$, $-COR^f$, $COOR^g$ ou un aryle;

$R^f$ est le reste d'un acide $\alpha$-aminé lié par le groupe amino ou de l'un de ses esters ou de ses amides;

$R^g$ est H ou un alkyle inférieur;

$R^2$ est H, $CH_3$, $OCH_3$, $NO_2$, un halogène, $NH_2$, NHCO-phénylène-COOH, $-NHCO(CH_2)_{1-4}-COOH$, $OR^h$, $-CH_2CH_2OR^h$, $-CH_2CH_2OCH_2CH_2OR^h$ ou $-CH_2COOR^h$; et

$R^h$ est H ou un alkyle inférieur,

ainsi que leurs hydrates ou solvates et leurs sels physiologiquement utilisables, caractérisé en ce que l'on clive sur un composé de formule

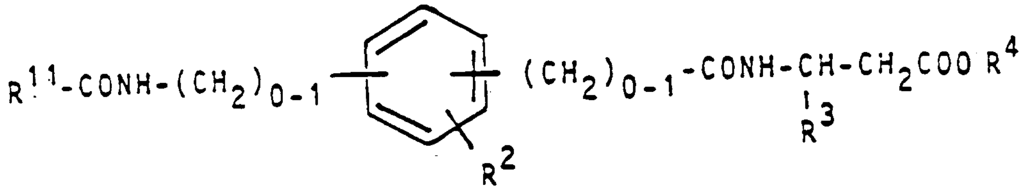 II

où $R^{11}$ représente un reste de formules R-1, R-2, R-3, R-4 ou R-5 conformes aux définitions données ci-dessus, dans laquelle un groupe amino, amidino ou guanidino éventuellement présent peut se présenter sous forme protégée, $R^4$ est l'hydrogène ou un groupe ester facilement clivable et $R^2$ et $R^3$ ont la signification donnée ci-dessus et où la molécule contient au moins un groupe ester facilement

clivable ou un groupe amino, amidino ou guanidino protégé, le ou les groupe(s) ester(s) et/ou le ou les groupe(s) protecteur(s) de l'amino, de l'amidino ou du guanidino, en ce que, si désiré, l'on modifie fonctionnellement un groupe réactif contenu dans le reste $R^1$ et, en ce que, si désiré, l'on transforme un composé de formule I en un sel physiologiquement compatible ou un sel d'un composé de formule I en un acide ou en une base libre.

2. Procédé selon la revendication 1 de formule

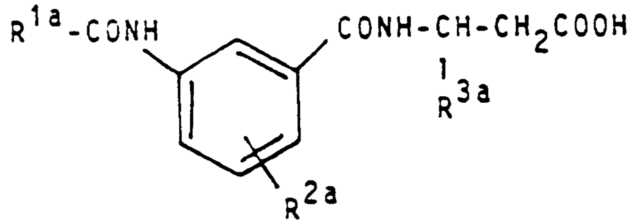

IA

où $R^{1a}$ est un groupe de formules R-1, R-2 ou R-3;
$R^{2a}$ est H, $-CH_3$, $-OCH_3$, $-NO_2$, un halogène, $-NH_2$, -NHCO-phénylène-COOH ou $-NHCO(CH_2)_{1-4}$-COOH;
$R^{3a}$ est H, $-CONH_2$ ou $-COR^1$;
$R^i$ est le reste d'un acide $\alpha$-aminé lié par le groupe amino
et R-1, R-2 et R-3 ont les significations données ci-dessus.

3. Procédé selon la revendication 1, où $R^1$ est un reste de formule R-1 ayant la signification donnée dans la revendication 1.

4. Procédé selon la revendication 3, où $R^1$ est un (amino ou guanidino)-$(CH_2)_{1-7}$- ou où $R^1$-CO est le reste acide de l'arginine, de l'ornithine, de la $N^2$-Boc-arginine ou de la $N^2$-Boc-ornithine.

5. Procédé selon la revendication 1, où $R^1$ est un reste R-2 ayant la signification donnée dans la revendication 1.

6. Procédé selon la revendication 5, où $R^1$ est un $\alpha$-(amino ou guanidino)-(m ou p)-tolyle ou l'$\alpha$-amino-p-tolylméthyle.

7. Procédé selon la revendication 1, où $R^1$ est un reste R-3 ayant la signification donnée dans la revendication 1.

8. Procédé selon la revendication 7, où $R^1$-CO est le 3-(p-amidinophényl)-DL-alanyle ou le p-amidinobenzoyle.

9. Procédé selon la revendication 1, où $R^1$ est un reste R-4.

10. Procédé selon la revendication 1, où $R^1$ est un reste R-5.

11. Procédé selon l'une des revendications 1 à 10, où $R^3$ est l'hydrogène, un carboxyisobutylidèneaminocarbonyle, le 2-carboxyphénylaminocarbonyle ou le 1-carboxy-2-(1-naphtyl)éthylidèneaminocarbonyle.

12. Procédé selon l'une des revendications 1 à 11, où $R^2$ est H, $-CH_3$, $-OCH_3$, $NO_2$ ou -Cl.

13. Procédé selon la revendication 3, dans lequel on prépare les composés selon la revendication 3 provenant du groupe des composés suivants :
la N-[m-(L-arginylamino)benzoyl]-$\beta$-alanine;
le formiate de N-[m-(L-arginylamino)benzoyl]-$\beta$-alanine;
le formiate de N-[3-(L-arginylamino)-p-anisoyl]-$\beta$-alanine;

le formiate de N-[3-(L-arginylamino)-p-toluoyl]-β-alanine;
la N-[m-(6-aminohexanamido)benzoyl]-β-alanine;
la N-[m-(β-alanylamino)benzoyl]-β-alanine;
la N-[m-(8 -aminooctanamido)benzoyl]-β-alanine;
la N-[m-(5-aminovaléramido)benzoyl]-β-alanine;
la N-[m-(6-guanidinohexanamido)benzoyl]-β-alanine;
la N-[m-(8-guanidinooctanamido)benzoyl]-β-alanine;
la N-[m-[(N-amidino-β-alanyl)amino]benzoyl]-β-alanine;
l'acétate de N-[N-[m-(L-arginylamino)benzoyl]-L-α-aspartyl]-L-valine;
le formiate de N-[3-(6-aminohexanamido)-5-nitrobenzoyl]-β-alanine;
la N-[5-(6-aminohexanamido)-2-chlorobenzoyl]-β-alanine;
la N-[5-(β-alanylamino)-2-nitrobenzoyl]-β-alanine;
la N-[3-(6-guanidinohexanamido)-5-nitrobenzoyl]-β-alanine;
la N-[5-(6-guanidinohexananido)-2-chlorobenzoyl]-β-alanine;
le [m-(Boc-Arg-NH)benzoyl]-Asp-Val;
la N-[m-(7-aminoheptanamido)benzoyl]-β-alanine;
la N-[m-(5-guanidinovaléramido)benzoyl]-β-alanine;
la N-[m-(7-guanidinoheptanamido)benzoyl]-β-alanine;
la N-[m-(4-guanidinobutyramido)benzoyl]-β-alanine;
la N-[m-(4-aminobutyramido)benzoyl]-β-alanine;
l'acétate de N-[m-[[(S)-$N_2$-(t-butoxycarbonyl)-L-ornithyl] amino]benzoyl]-β-alanine;
le formiate de N-[m-(L-ornithylamino)benzoyl]-β-alanine;
le trifluoroacétate de N-[5-(β-alanylamino)anthraninoyl] -β-alanine;
le formiate de N-[m-[(N-amidinoglycyl)amino]benzoyl]-β-alanine;
le formiate de N-[m-(L-lysylamino)benzoyl]-β-alanine;
le formiate de DL-N-[m-(5-aminovaléramido)benzoyl]-3-phényl-β-alanine;
le formiate de N-[3-(5-aminovaléramido)-4-chlorobenzoyl]-β-alanine;
la N-[3-(5-aminovaléramido)-p-toluoyl]-β-alanine;
le trifluoroacétate de DL-N-[m-(5-aminovaléramido)benzoyl] -3-(5-indanyl)-β-alanine;
le formiate de DL-N-[m-(5-guanidinovaléramido)benzoyl]-3-(5-indanyl)-β-alanine;
la N-[5-(5-aminovaléramido)-2-benzyloxy)benzoyl]-β-alanine;
la N-[2-(benzyloxy)-5-(5-guanidinovaléramido)benzoyl]-β-alanine;
la N-[5-(5-guanidinovaléramido)salicyloyl]-β-alanine.

**14.** Procédé selon la revendication 5, dans lequel on prépare les composés selon la revendication 5 provenant du groupe des composés suivants :
la N-[m-(α-amino-p-toluamido)benzoyl]-β-alanine;
la N-[m-(α-guanidino-p-toluamido)benzoyl]-β-alanine;
la N-[m-(α-amino-m-toluamido)benzoyl]-β-alanine;
la N-[m-(α-guanidino-m-toluamido)benzoyl]-β-alanine;
la N-[m-[2-(α-amino-p-tolyl)acétamido]benzoyl]-β-alanine;
le trifluoroacétate de N-[N-[m-[2-(p-amidinophényl) acétamido]benzoyl]-L-α-aspartyl]-3-phényl-L-alanine;
la N-[[m-(α-amino-p-toluamido)phényl]acétyl]-β-alanine.

**15.** Procédé selon la revendication 7, dans lequel on prépare les composés selon la revendication 7 provenant du groupe des composés suivants :
l'acétate de N-[m-[[3-(p-amidinophényl)-DL-alanyl]amino] benzoyl]-β-alanine;
le trifluoroacétate de N-[N-[m-[[3-(p-amidinophényl)-DL-alanyl]amino]benzoyl]-L-α-aspartyl]-3-(1-naphtyl)-L-alanine;
le trifluoroacétate de N-[N-[m-(p-amidinobenzamido)benzoyl] -L-α-aspartyl]-3-(1-naphtyl)-L-alanine;
la N-[m-(p-amidinobenzamido)benzoyl]-β-alanine;
l'iodhydrate de l'ester méthylique de la N-[m-(p-amidinobenzamido)benzoyl]-3-(tert-butoxycarbonyl)-L-alanine;
le trifluoroacétate de l'acide (S)-3-[m-(p-amidinobenzamido)benzamido]-3-(méthoxycarbonyl)-propionique;
la N-[m-(p-amidinobenzamido)benzoyl]-3-(tert-butoxycarbonyl)-L-alanine;
le trifluoracétate de N-[m-(p-amidinobenzamido)benzoyl]-L-asparagine;
l'acide (S)-3-[m-(p-amidinobenzamido)benzamido]-3-(aminocarbonyl)propionique;

la N-[m-(m-amidinobenzamido)benzoyl]-β-alanine;
la N-[α-(p-amidinobenzamido)-m-toluoyl]-β-alanine;
la N-[α-(p-amidinobenzamido)-p-toluoyl]-β-alanine;
la N-[m-[2-(p-amidinophényl)acétamido]benzoyl]-β-alanine;
la N-[5-(p-amidinobenzamido)-2-(benzyloxy)benzoyl]-β-alanine;
la N-[3-(p-amidinobenzamido)-4-(benzyloxy)benzoyl]-β-alanine;
la N-[3-(p-amidinobenzamido)-4-hydroxybenzoyl]-β-alanine;
la N-[5-(p-amidinobenzamido)salicyloyl]-β-alanine;
la N-[p-(p-amidinobenzamido)benzoyl]-β-alanine.

**16.** Procédé selon la revendication 9, dans lequel on prépare la N-[m-[trans-4-(aminométhyl)-cyclohexanecarboxamido]benzoyl]-β-alanine.

**17.** Procédé selon la revendication 10, dans lequel on prépare les composés selon la revendication 10 provenant du groupe des composés suivants :
le trifluoroacétate de N-[N-[m-(1-amidino-4-pipéridinecarboxamido)benzoyl]-L-α-aspartyl]-3-phényl-L-alanine;
la N-[m-[3-(4-pipéridinyl)propionamido)benzoyl]-β-alanine;
le trifluoroacétate de N-[m-[3-(1-amidino-4-pipéridinyl) propionamido]benzoyl]-β-alanine.

**18.** Procédé selon la revendication 1, dans lequel on saponifie un groupe ester avec une base, on clive un ester benzylique par hydrogénation catalytique, on clive un groupe amino protégé avec un acide à une température allant jusqu'à 40 ° C.

**19.** Procédé pour la préparation de compositions pharmaceutiques, caractérisé en ce que l'on met un composé de formule I selon la revendication 1 ou l'un de ses sels physiologiquement compatibles sous forme d'une présentation galénique.

**20.** Utilisation d'un composé de formule I de la revendication 1 pour la préparation de médicaments destinés au traitement ou à la prévention des thromboses, des attaques cérébrales, des infarctus du myocarde, de l'artériosclérose, des inflammations et des carcinomes.

**21.** Composés de formule

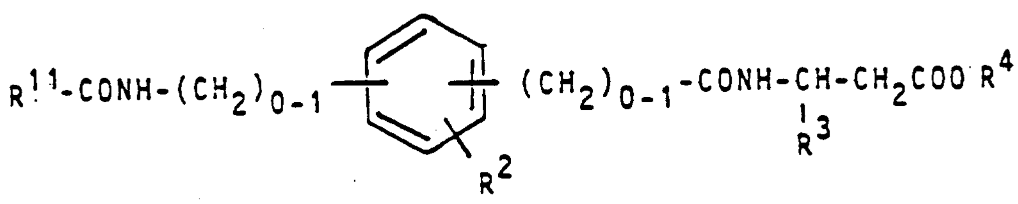

II

où $R^{11}$ représente un reste de formules R-1, R-2, R-3, R-4 ou R-5 conformes aux définitions données dans la revendication 1, dans laquelle un groupe amino, amidino ou guanidino éventuellement présent peut se présenter sous forme protégée, $R^4$ est l'hydrogène ou un groupe ester facilement clivable et $R^2$ et $R^3$ ont les significations données dans la revendication 1; et où la molécule contient au moins un groupe ester facilement clivable ou un groupe amino, amidino ou guanidino protégé.